# EUROPEAN PATENT APPLICATION

(11) **EP 4 621 070 A1**
(43) Date of publication of application: **24.09.2025**
(21) Application number: 24382299.6
(22) Date of filing: 20.03.2024
(51) Int. Cl.: C12Q 1/6883, G01N 33/52

(54) **IN VITRO METHODS FOR THE DIAGNOSIS OF ALLERGIC ASTHMA OR NONALLERGIC ASTHMA IN A SUBJECT SUFFERING FROM ASTHMA, OR FOR THE PROGNOSIS OF ASTHMA**

(71) Applicant: Fundación Instituto de Investigación Sanitaria Fundación Jiménez Díaz, 28040 Madrid (ES); Consorcio Centro de Investigación Biomédica en Red, 28029 Madrid (ES)
(72) Inventor: CÁRDABA OLOMBRADA, Blanca, 28040 Madrid (ES); CREMADES JIMENO, Lucía, 28040 Madrid (ES); BAOS MUÑIZ, Selene, 28040 Madrid (ES); LÓPEZ RAMOS, María, 28040 Madrid (ES); MAHILLO FERNÁNDEZ, Ignacio, 28040 Madrid (ES); DE PEDRO MUNOZ, Mª Ángeles, 28040 Madrid (ES)
(74) Representative: Hoffmann Eitle

(57) **Abstract**

The present invention refers to the medical field. Particularly, the present invention refers to an *in vitro* method for the diagnosis of allergic asthma or nonallergic asthma in a subject suffering from asthma, or for the differential diagnosis of allergic asthma and nonallergic asthma in a subject suffering from asthma, or for the prognosis of asthma in a subject suffering from asthma, which comprises assessing the expression level of the *LGALS3* gene in a biological sample obtained from the subject. It also refers to the *in vitro* use of the expression level of the *LGALS3* gene, or *in vitro* use of a kit comprising reagents for the same purposes.

## Description

### FIELD OF THE INVENTION

The present invention refers to the medical field. Particularly, the present invention refers to an *in vitro* method for the diagnosis of allergic asthma or nonallergic asthma in a subject suffering from asthma, or for the differential diagnosis of allergic asthma and nonallergic asthma in a subject suffering from asthma, or for the prognosis of asthma in a subject suffering from asthma, which comprises assessing the expression level of the *LGALS3* gene in a biological sample obtained from the subject. It also refers to the *in vitro* use of the expression level of the *LGALS3* gene, or *in vitro* use of a kit comprising reagents for the same purposes.

### STATE OF THE ART

Chronic inflammatory respiratory diseases, including allergic diseases and asthma, are common, complex and heterogeneous diseases, which is why both their clinical evolution and their treatment are not always predictable.

Allergic diseases are adverse reactions of the immune system to theoretically harmless substances whose prevalence is not only very high worldwide, but is increasing, albeit in a lifestyle-dependent and variable manner, reflecting their multifactorial nature. As highlighted by the European Academy of Allergy and Clinical Immunology (EAACI), allergy is today a public health problem of pandemic proportions, affecting more than 150 million people in Europe; in fact, it is the most common chronic disease. Based on epidemiological trends, EAACI estimates that more than half of the European population will suffer from some form of allergy by 2025. This high prevalence and its impact on quality of life make it a serious problem for the economy (lower productivity of those affected and higher absenteeism from work) and for public health systems. It is estimated that the most common clinical manifestations of these diseases, asthma and rhinitis, account for more than 100 million days of absenteeism from work or school per year.

Bronchial asthma is an inflammatory disease of the airways that causes bronchial hyperresponsiveness and/or airflow obstruction characterized by symptoms such as cough, wheezing or dyspnoea. The World Health Organization (WHO) defines it as the most common chronic disease in children and it is estimated that there are more than 300 million affected. Of these, approximately 10% show severe asthma symptoms, with significant morbidity and mortality. In the latest Global Initiative for Asthma (GINA) it was defined as "a heterogeneous disease, usually characterized by chronic inflammation of the airways." It is characterized by a history of respiratory symptoms such as wheezing, shortness of breath, chest tightness, and cough that vary in intensity over time and is associated with variable expiratory airflow limitation. However, one of the main problems in defining this pathology is its wide clinical spectrum, from an occasional episode (which can be easily reversed) to a sustained blockage that requires high doses of oral or inhaled corticosteroids. In the medical community, it is generally accepted that clinical differences observed in responses to treatment or in the course of the disease over time are related to multiple underlying variations in genetic, pharmacological, physiological, biological and /or immunological that produce subclasses of phenotypes referred to as endotypes. This heterogeneity has led to calls for the pursuit of precision or personalized medicine (among others). For this reason, more and more groups speak of endotypes or various forms of the disease, requiring different diagnostic and therapeutic approaches, as recently reviewed by experts in this area.

Probably due to this great complexity and heterogeneity, to date, there is no cure for asthma. The main objective of asthma treatment is to achieve and maintain control of the disease as early as possible, as well as to prevent exacerbations and chronic airflow obstruction and minimizing mortality. The objectives of treatment, both in terms of controlling daily symptoms (current control domain) and preventing exacerbations and an exaggerated loss of lung function (future risk domain), can be achieved in a high percentage of patients with adequate treatment, but there is always a proportion that does not respond adequately to any treatment.

Multiple studies have characterized various phenotypes of the disease in certain groups of patients with recognizable demographic, clinical or pathophysiological characteristics. However, although in patients with severe uncontrolled asthma, such classification may be helpful in guiding specific treatments, at the moment there is no robust evidence to recommend a classification of the disease based on asthma phenotypes in general, and in which controlled with usual treatment, in particular. Asthma phenotypes can be grouped into three large blocks (not mutually exclusive): clinical or physiological, trigger-related, and inflammatory **(Table 1).**

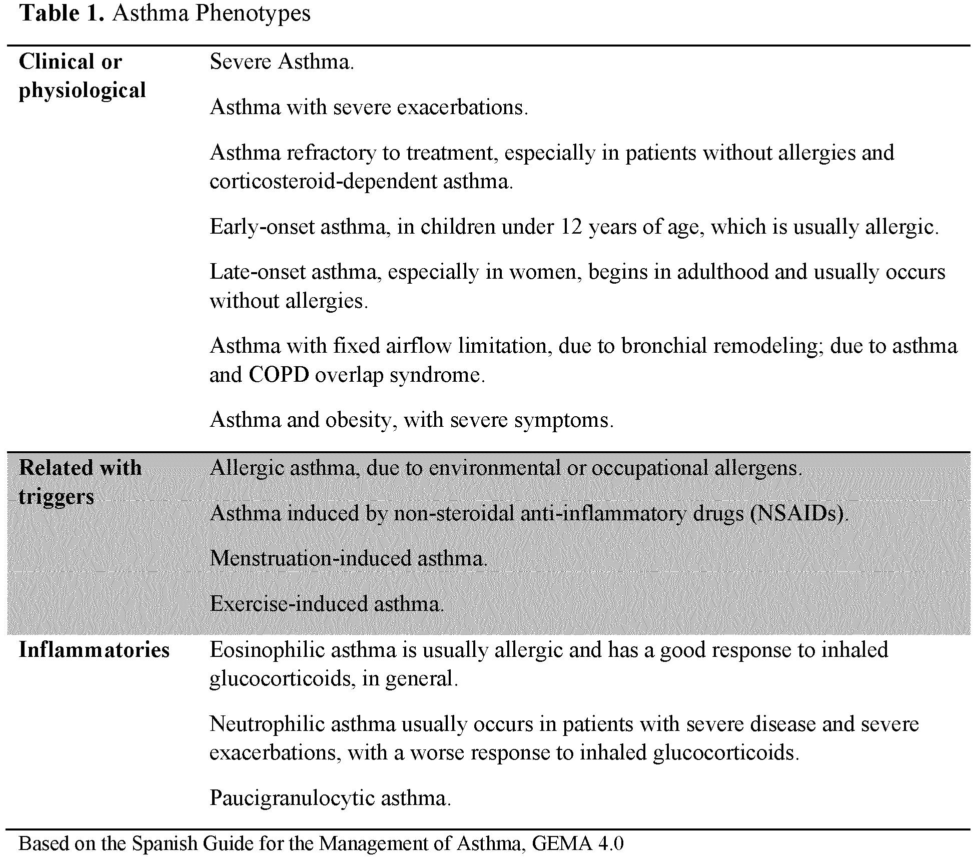

### Classification of adult asthma and clinical severity

Asthma has typically been classified according to severity, although the definition has evolved over time. Asthma severity is an intrinsic property of the disease, reflecting the severity of pathophysiological abnormalities. It must be taken into account that the severity of asthma implies both the intensity of the process and the response to treatment. Severity is usually assessed when the patient is being treated and is classified according to the maintenance treatment needs required to achieve control of symptoms and exacerbations. It is traditionally divided into four categories: intermittent, mild persistent, moderate persistent and severe persistent **(Table 2).**

Severity is not necessarily a constant characteristic of asthma, but it can vary over time (months or years), so it needs to be reassessed periodically. Severity is determined retrospectively in the patient whose asthma is controlled according to the therapeutic step in which they are located, that is, based on the amount of medication that is necessary to maintain control of the disease, resorting to reducing the step if necessary to stipulate the minimum amounts of treatment. It can be established in a patient who is not receiving maintenance treatment, but this is rare.

### Control

Asthma control is the degree to which asthma manifestations are absent or minimized by therapeutic interventions and treatment goals are met. Control largely reflects the adequacy of asthma therapy. However, another factor, which differs from patient to patient, is the response to treatment and the ease and speed with which control is achieved. Although the term control is broad and can encompass all clinical and pathophysiological aspects of asthma, for practical purposes it includes the clinical features of the disease (symptoms and exacerbations) and lung function tests.

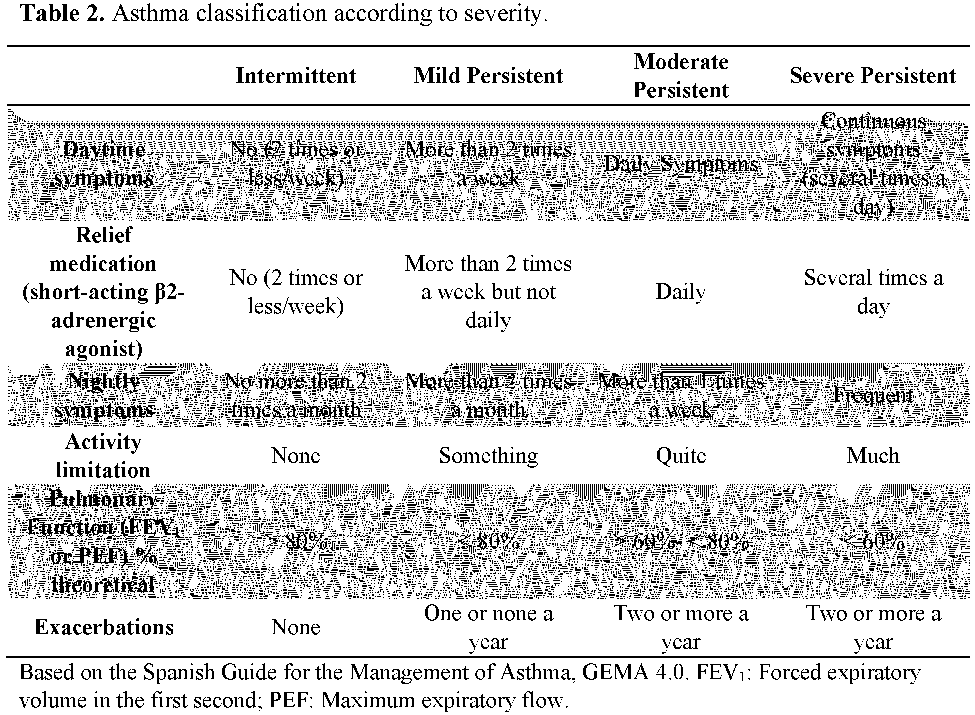

Drugs to treat asthma are classified as control or maintenance, and relief, also called "rescue." Control or maintenance medications, which should be administered daily for prolonged periods, include inhaled glucocorticoids (IGC) or systemic glucocorticoids, leukotriene receptor antagonists (LRTA), long-acting β2-adrenergic agonists (LABA), tiotropium, and antibodies. anti-IgE monoclonals (omalizumab). Chromones and delayed-release theophylline have fallen out of use due to their lower effectiveness. Reliever medications are used as needed to quickly treat or prevent bronchoconstriction and include inhaled short-acting β2-adrenergic agonists (SABA) (of choice) and inhaled anticholinergics (ipratropium bromide).

Severe asthma is characterized by the need for multiple drugs and high doses of treatment. It is associated with a greater consumption of economic resources compared to moderate or mild asthma. Severe uncontrolled asthma (SCNA) is defined as asthmatic disease that persists poorly controlled despite receiving treatment with a combination of GCI/LABA, at high doses in the last year, or oral glucocorticoids for at least six months of the same period.

### Technical problems solved by the present invention

As previously described, from a clinical point of view, asthma is a very heterogeneous disease, with a large number of different phenotypes. However, despite this clinical heterogeneity of asthma, allergic mechanisms have been implicated in 50-80% of asthmatic patients and in approximately 50% of severe asthma. This is one of the reasons why asthma has been commonly associated with type 2 (Th2) respiratory inflammation, characterized by high levels of IgE, eosinophils and some cytokines such as IL4, IL5, IL13 and IL9, canonically associated with allergic responses and for that reason, a large part of the efforts in the search for new treatments for asthma have focused on the Th2 cytokine pathway. However, the type 2 immune response is a complex endotype, with several sub-endotypes, such as endotypes defined as IL-5-high, IL-13-high or IgE-high that define subgroups of patients that would have therapeutic benefits with different treatment targets. New strategies for the discovery and validation of molecular biomarkers such as omic approaches have been used to reveal the mechanisms responsible for asthma endotypes in different tissues. A biomarker is defined as an objective, measurable parameter that may be the signature of a complex underlying pathway or a key molecule associated with, or directly plays an essential role in, a particular disease endotype. Several new experimental treatments, known as biologic therapies, are in various stages of clinical development for patients with inflammation driven by a type 2 immune response: anti-IL-4/IL-13, anti-IL-4, anti-IL antibodies -5 and anti-IgE, as well as CRTH2 (chemokine receptor homolog of a molecule expressed on Th2 lymphocytes). A summary of the status of these biological therapies at present is outlined in **Tables 3** and **4.** However, at present, the available biomarkers are not specific enough to select the type 2 immune response asthma subendotype that specifically responds to targeted treatment. This may be due to several factors, such as genetic (or epigenetic) influence or lack of knowledge of the predominant inflammatory immune pathway or the contribution of the remodeled tissue itself to the response. In summary, although recent therapeutic advances have unravelled some of the contributions of different phenotypes and endotypes to the pathogenesis of asthma and responses to specific therapies, more information is still needed to optimise the patient's therapeutic responses while trying to avoid adverse effects.

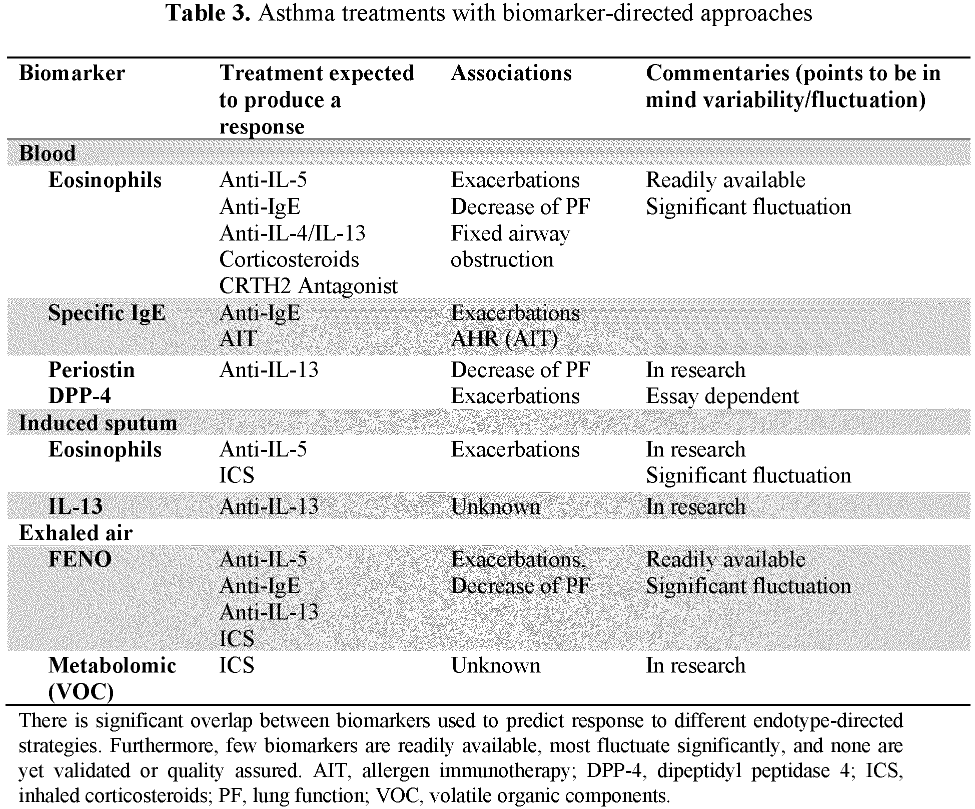

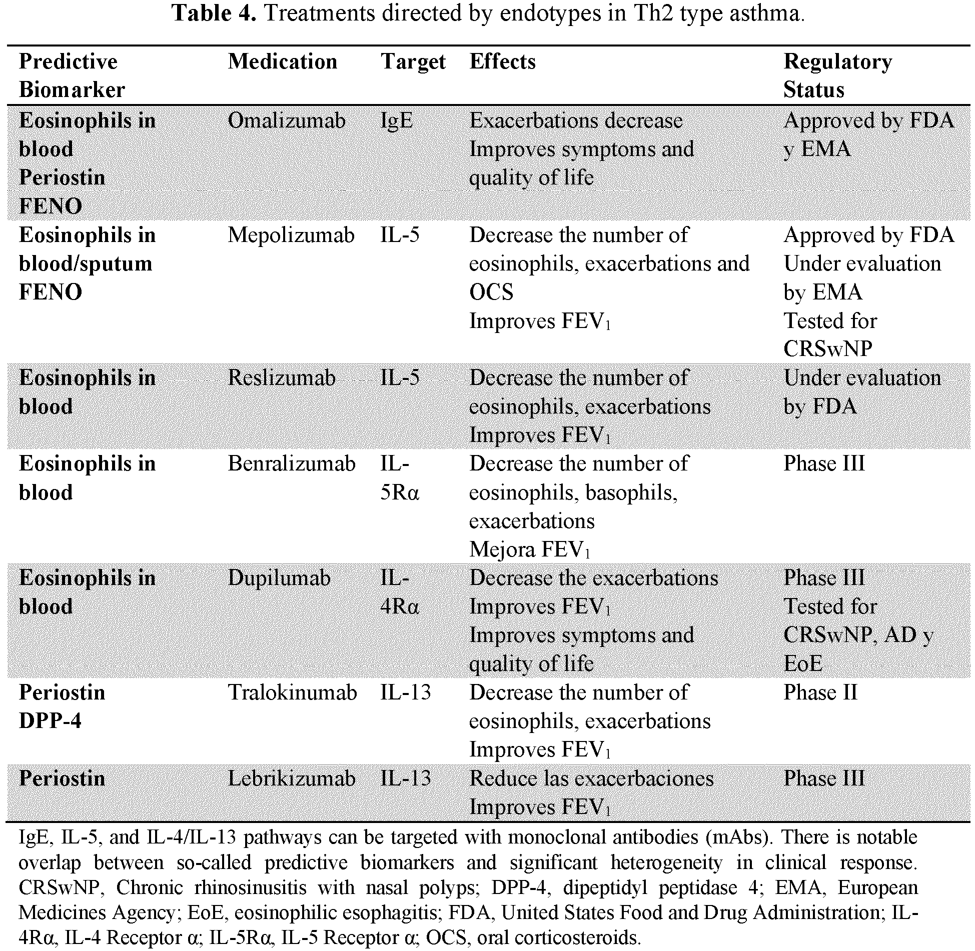

On the other hand, although allergic asthma (AA) affects a significant proportion of patients, from 10% to 33% of subjects with asthma are nonallergic (NA) subjects, defined as subjects with asthma but without any associated allergic sensitization. In these subjects, the mechanisms contributing to the type 2 immune response are less clear. In many cases, instead of eosinophilic inflammation, there is a prevalence of neutrophils. The non-Th2-mediated immune response endotype of asthma is much less known than the Th2 type and until now no therapies directed against this endotype have been demonstrated to be effective. Therefore, efforts directed toward this type of asthma are clearly an unmet need and biologically targeted therapies are a field to be developed.

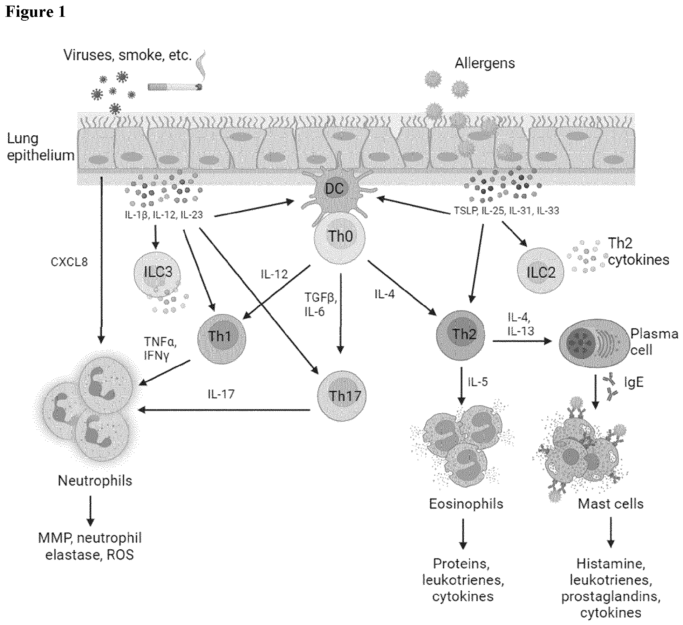

Asthmatic inflammation can be classified into two endotypes: type 2 (T2), or eosinophilic asthma, which can in turn be of allergic or nonallergic origin; and non-T2 (or T2 low), or non-eosinophilic asthma. The cells, molecules and mechanisms involved in each type of inflammation are different, as can be seen in **Figure 1****,** and these differences are the cause of the different response to treatment observed in patients.

The therapeutic approach to this disease is very complex and, in most cases, treatments are necessary for long periods of time, even for life. The drugs used to treat asthma can be classified as: maintenance medication, which is administered continuously with the aim of reducing inflammation and preventing the appearance of symptoms; and rescue medication, which are used as needed to quickly treat or prevent bronchoconstriction. In the most severe cases of asthma, biological treatment with monoclonal antibodies can be considered, which must be chosen based on the patient's endotype, which is why the correct diagnosis is essential. However, there are currently no reliable and well-defined diagnostic biomarkers for asthma, delegating both the diagnosis of asthma and the classification of its severity mainly to symptoms and response to treatment.

For all these reasons, there is an unmet medical need for the identification of biomarkers for the diagnosis and prognosis of asthma, and for the development of a method for the diagnosis and prognosis of asthma. The present invention is focused on solving this problem and an *in vitro* method for the diagnosis of allergic asthma or nonallergic asthma in a subject suffering from asthma, or for the differential diagnosis of allergic asthma and nonallergic asthma in a subject suffering from asthma, or for the prognosis of asthma in a subject suffering from asthma, is herein provided.

### DESCRIPTION OF THE INVENTION

### Brief description of the invention

As explained above, the present invention refers to an *in vitro* method for the diagnosis of allergic asthma or nonallergic asthma in a subject suffering from asthma, or for the differential diagnosis of allergic asthma and nonallergic asthma in a subject suffering from asthma, or for the prognosis of asthma in a subject suffering from asthma, which comprises assessing the expression level of the *LGALS3* gene in a biological sample obtained from the subject. It also refers to the *in vitro* use of the expression level of the *LGALS3* gene, or *in vitro* use of a kit comprising reagents for the same purposes.

Using computational analyses, the inventors have identified several genetic biomarkers that can be used for the diagnosis or prognosis of different types of asthma. These biomarkers may be used:
- for the diagnosis of nonallergic asthma **(Table 7A, 9A** and **10A),** allergic asthma **(Table 7B, 9B** and **10B),** mild nonallergic asthma **(Table 8A),** moderate nonallergic asthma **(Table 8A),** severe nonallergic asthma **(Table 8A),** mild allergic asthma **(Table 8B),** moderate allergic asthma **(Table 8B)** and severe allergic asthma **(Table 8A);** and
- for the differential diagnosis of allergic and nonallergic asthma **(Table 10C),** mild and moderate allergic asthma **(Table 11A** and **12A),** mild and severe allergic asthma **(Table 11A** and **12A),** moderate and severe allergic asthma **(Table 11A** and **12A),** mild and moderate nonallergic asthma **(Table 11B** and **12B),** mild and severe nonallergic asthma **(Table 11B** and **12B),** and moderate and severe nonallergic asthma **(Table 11B** and **12B).**

These biomarkers are based on the results of a longitudinal analysis performed with samples from asthmatic (allergic and nonallergic) and healthy subjects. Samples from peripheral blood mononuclear cells (PBMCs) were collected at two different timepoints: at the moment of inclusion in the study and at two years after inclusion.

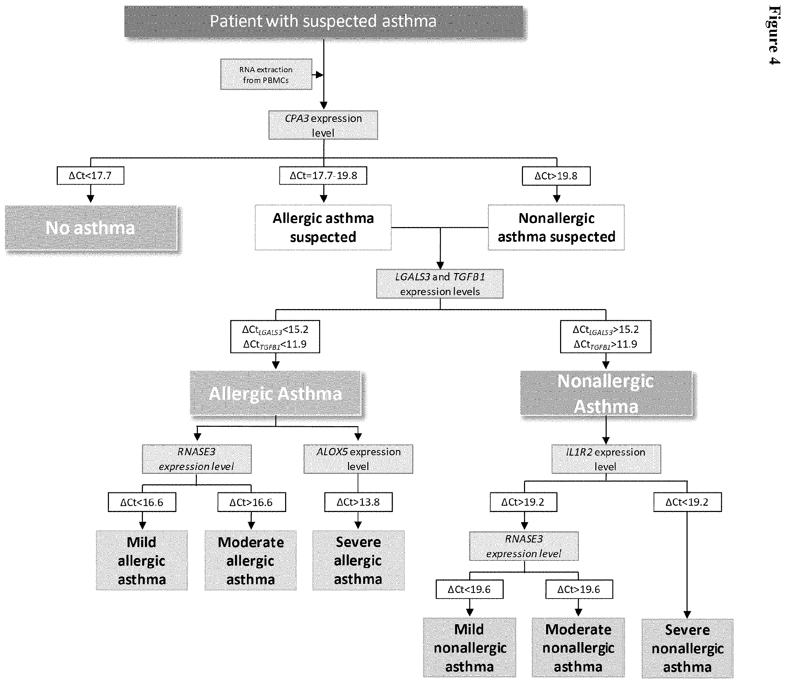

In addition, the inventors have developed an algorithm for the differential diagnosis of different asthma phenotypes based on the detection of six of these genetic biomarkers **(****Figure 4****).**

The first embodiment of the invention refers to an *in vitro* method for the diagnosis of allergic asthma or nonallergic asthma in a subject suffering from asthma, or for the differential diagnosis of allergic asthma and nonallergic asthma in a subject suffering from asthma, or for the prognosis of asthma in a subject suffering from asthma, which comprises:
a) assessing the expression level of the *LGALS3* gene in a biological sample obtained from the subject,
b) wherein an increased expression level of the *LGALS3* gene, as compared to a pre-established reference value, is indicative that the subject suffers from allergic asthma, and/or
c) wherein a decreased expression level of the *LGALS3* gene, as compared to a pre-established reference value, is indicative that the subject suffers from nonallergic asthma.

In a preferred embodiment, the *in vitro* method comprises:
a) assessing the expression level of the *LGALS3* gene in combination with a gene selected from *TGFB1*, *ALOX5*, *BAX*, *CCL5*, *CD40*, *CHI3L1*, *CPA3*, *IL1R2*, *IL4R*, *PI3*, *RNASE3* or *SELL* in a biological sample obtained from the subject,
b) wherein an increased expression level of the *LGALS3* gene and of a gene selected from *TGFB1*, *ALOX5*, *BAX*, *CCL5*, *CD40*, *CHI3L1*, *CPA3*, *IL1R2*, *IL4R*, *PI3*, *RNASE3* or *SELL*, as compared to a pre-established reference value, is indicative that the subject suffers from allergic asthma, and/or
c) wherein a decreased expression level of the *LGALS3* gene and of a gene selected from *TGFB1*, *ALOX5*, *BAX*, *CCL5*, *CD40*, *CHI3L1*, *CPA3*, *IL1R2*, *IL4R*, *PI3*, *RNASE3* or *SELL*, as compared to a pre-established reference value, is indicative that the subject suffers from nonallergic asthma.

In a preferred embodiment, the *in vitro* method comprises:
a) assessing the expression level of the genes *LGALS3* and *TGFB1* in a biological sample obtained from the subject,
b) wherein an increased expression level of the genes *LGALS3* and *TGFB1,* as compared to a pre-established reference value, is indicative that the subject suffers from allergic asthma, and/or
c) wherein a decreased expression level of the genes *LGALS3* and *TGFB1,* as compared to a pre-established reference value, is indicative that the subject suffers from nonallergic asthma.

In a preferred embodiment, the *in vitro* method comprises:
a) assessing the expression level of the genes *LGALS3* and *TGFB1* in combination with a gene selected from *ALOX5*, *BAX*, *CCL5*, *CD40*, *CHI3L1*, *CPA3*, *IL1R2*, *IL4R*, *PI3*, *RNASE3* or *SELL* in a biological sample obtained from the subject,
b) wherein an increased expression level of the genes *LGALS3*, *TGFB1* and a gene selected from *ALOX5*, *BAX*, *CCL5*, *CD40*, *CHI3L1*, *CPA3*, *IL1R2*, *IL4R*, *PI3*, *RNASE3* or *SELL*, as compared to a pre-established reference value, is indicative that the subject suffers from allergic asthma, and/or
c) wherein a decreased expression level of the genes *LGALS3*, *TGFB1* and a gene selected from *ALOX5*, *BAX*, *CCL5*, *CD40*, *CHI3L1*, *CPA3*, *IL1R2*, *IL4R*, *PI3*, *RNASE3* or *SELL*, as compared to a pre-established reference value, is indicative that the subject suffers from nonallergic asthma.

A preferred embodiment of the invention refers to an *in vitro* method is for the prognosis of allergic asthma or for the differential diagnosis of mild or moderate allergic asthma and severe allergic asthma, which further comprises:
a) assessing the expression level of the *ALOX5* gene, in a biological sample obtained from the subject,
b) wherein an increased expression level of the *ALOX5* gene, as compared to a pre-established reference value, is indicative that the subject suffers from mild or moderate allergic asthma, and/or
c) wherein a decreased expression level of the *ALOX5* gene, as compared to a pre-established reference value, is indicative that the subject suffers from severe allergic asthma.

In preferred embodiment the *in vitro* method further comprises:
a) assessing the expression level of one or more of the genes *BAX, CCL5, CD40, CHI3L1, CPA3,* IL4R, *LGALS3, PI3, RNASE3, SELL* or *TGFB1* in a biological sample obtained from the subject,
b) wherein an increased expression level of the genes *BAX*, *CCL5*, *CD40*, *CPA3*, IL4R, *LGALS3*, *RNASE3*, *SELL* or *TGFB1*, and/or a decreased expression level of the genes *CHI3L1* or *PI3*, as compared to a pre-established reference value, is indicative that the subject suffers from mild or moderate allergic asthma, and/or
c) wherein a decreased expression level of the genes *BAX*, *CCL5*, *CD40*, *CPA3*, IL4R, *LGALS3*, *RNASE3*, *SELL* or *TGFB1*, and/or an increased expression level of the genes *CHI3L1* or *PI3*, as compared to a pre-established reference value, is indicative that the subject suffers from severe allergic asthma.

A preferred embodiment of the invention refers to an *in vitro* method for the prognosis of allergic asthma or for the differential diagnosis of mild allergic asthma and moderate allergic asthma, which further comprises:
a) assessing the expression level of the *RNASE3* gene in a biological sample obtained from the subject,
b) wherein an increased expression level of the *RNASE3* gene, as compared to a pre-established reference value, is indicative that the subject suffers from mild allergic asthma, and/or
c) wherein a decreased expression level of the *RNASE3* gene, as compared to a pre-established reference value, is indicative that the subject suffers from moderate allergic asthma.

In preferred embodiment the *in vitro* method further comprises:
a) assessing the expression level of one or more of the genes *ALOX5*, *BAX*, *CCL5*, *CD40*, *CHI3L1*, *CPA3*, *IL1R2*, *IL4R*, *LGALS3*, *PI3*, *SELL* or *TGFB1* in a biological sample obtained from the subject,
b) wherein an increased expression level of the genes *ALOX5*, *BAX*, *CCL5*, *CD40*, *CHI3L1*, *CPA3*, *IL1R2*, *IL4R*, *LGALS3*, *PI3*, *SELL* or *TGFB1*, as compared to a pre-established reference value, is indicative that the subject suffers from mild allergic asthma, and/or
c) wherein a decreased expression level of the genes *ALOX5*, *BAX*, *CCL5*, *CD40*, *CHI3L1*, *CPA3*, *IL1R2*, *IL4R*, *LGALS3*, *PI3*, *SELL* or *TGFB1*, as compared to a pre-established reference value, is indicative that the subject suffers from moderate allergic asthma.

A preferred embodiment of the invention refers to an *in vitro* method for the prognosis of nonallergic asthma or for the differential diagnosis of mild or moderate nonallergic asthma and severe nonallergic asthma, which comprises:
a) assessing the expression level of the *IL1R2* gene in a biological sample obtained from the subject,
b) wherein a decreased expression level of the *IL1R2* gene, as compared to a pre-established reference value, is indicative that the subject suffers from mild or moderate nonallergic asthma, and/or
c) wherein an increased expression level of the *IL1R2* gene, as compared to a pre-established reference value, is indicative that the subject suffers from severe nonallergic asthma.

In preferred embodiment the *in vitro* method further comprises:
a) assessing the expression level of one or more of the genes *ALOX5*, *BAX*, *CCL5*, *CD40*, *CHI3L1*, *IL4R*, *PI3*, *RNASE3* or *SELL* in a biological sample obtained from the subj ect,
b) wherein an increased expression level of the genes *CCL5*, *CD40* or *SELL*, and/or a decreased expression level of the genes *ALOX5*, *BAX*, *CHI3L1*, *IL4R*, *PI3* or *RNASE3*, as compared to a pre-established reference value, is indicative that the subject suffers from mild or moderate nonallergic asthma, and/or
c) wherein a decreased expression level of the genes *CCL5*, *CD40* or *SELL*, and/or an increased expression level of the genes *ALOX5*, *BAX*, *CHI3L1*, *IL4R*, *PI3* or *RNASE3*, as compared to a pre-established reference value, is indicative that the subject suffers from severe nonallergic asthma.

A preferred embodiment of the invention refers to an *in vitro* method for the prognosis of nonallergic asthma or for the differential diagnosis of mild nonallergic asthma and moderate nonallergic asthma, which further comprises:
a) assessing the expression level of the *RNASE3* gene in a biological sample obtained from the subject,
b) wherein an increased expression level of the *RNASE3* gene, as compared to a pre-established reference value, is indicative that the subject suffers from mild nonallergic asthma, and/or
c) wherein a decreased expression level of the *RNASE3* gene, as compared to a pre-established reference value, is indicative that the subject suffers from moderate nonallergic asthma.

In preferred embodiment the *in vitro* method further comprises:
a) assessing the expression level of one or more of the genes *ALOX5*, *BAX*, *CCL5*, *CD40*, *CHI3L1*, *CPA3*, *IL1R2*, *IL4R*, *LGALS3*, *PI3*, *SELL* or *TGFB1* in a biological sample obtained from the subject,
b) wherein an increased expression level of the genes *BAX*, *CD40*, *LGALS3*, *PI3* or *SELL,* and/or a decreased expression level of the genes *ALOX5*, *CCL5*, *CHI3L1*, *CPA3*, *IL1R2*, *IL4R* or *TGFB1*, as compared to a pre-established reference value, is indicative that the subject suffers from mild nonallergic asthma, and/or
c) wherein a decreased expression level of the *BAX*, *CD40*, *LGALS3*, *PI3* or *SELL*, and/or an increased expression level of the genes *ALOX5*, *CCL5*, *CHI3L1*, *CPA3*, *IL1R2*, *IL4R* or *TGFB1*, as compared to a pre-established reference value, is indicative that the subject suffers from moderate nonallergic asthma.

In preferred embodiment the subject is a mammal.

In preferred embodiment the *in vitro* the subject is a human.

In preferred embodiment the biological sample is peripheral blood mononuclear cells, blood, serum, plasma, bronchoalveolar lavage fluid, more preferably peripheral blood mononuclear cells.

The second embodiment of the invention refers to the *in vitro* use of the expression level of the *LGALS3* gene, or of the expression level of the *LGALS3* gene in combination with the expression level of a gene selected from *TGFB1*, *ALOX5*, *BAX*, *CCL5*, *CD40*, *CHI3L1*, *CPA3*, *IL1R2*, *IL4R*, *PI3*, *RNASE3* or *SELL*, or of the expression level of the genes *LGALS3* and *TGFB1*, or of the expression level of the genes *LGALS3* and *TGFB1* in combination with the expression level of a gene selected from *ALOX5*, *BAX*, *CCL5*, *CD40*, *CHI3L1*, *CPA3*, *IL1R2*, *IL4R*, *PI3*, *RNASE3* or *SELL*, for the diagnosis of allergic asthma or nonallergic asthma in a subject suffering from asthma, or for the differential diagnosis of allergic asthma and nonallergic asthma in a subject suffering from asthma, or for the prognosis of asthma in a subject suffering from asthma; or *in vitro* use of a kit comprising reagents for the assessment of the expression level of the *LGALS3* gene, or of the expression level of the *LGALS3* gene in combination with the expression level of a gene selected from *TGFB1*, *ALOX5*, *BAX*, *CCL5*, *CD40*, *CHI3L1*, *CPA3*, *IL1R2*, *IL4R*, *PI3*, *RNASE3* or *SELL*, or of the expression level of the genes *LGALS3* and *TGFB1*, *or* of the expression level of the genes *LGALS3* and *TGFB1* in combination with the expression level of a gene selected from *ALOX5*, *BAX*, *CCL5*, *CD40*, *CHI3L1*, *CPA3*, *IL1R2*, *IL4R*, *PI3*, *RNASE3* or *SELL*, for the diagnosis of allergic asthma or nonallergic asthma in a subject suffering from asthma, or for the differential diagnosis of allergic asthma and nonallergic asthma in a subject suffering from asthma, or for the prognosis of asthma in a subject suffering from asthma.

In a preferred embodiment, the *in vitro* use or *in vitro* use of a kit further comprises the expression level of the *ALOXS* gene or of the expression level of the *ALOXS* gene in combination with the expression level of one or more of the genes *BAX*, *CCL5*, *CD40*, *CHI3L1*, *CPA3*, *IL4R*, *LGALS3*, *PI3*, *RNASE3*, *SELL* and *TGFB1*, for the prognosis of allergic asthma or for the differential diagnosis of mild or moderate allergic asthma and severe allergic asthma.

In a preferred embodiment, the *in vitro* use or *in vitro* use of a kit further comprises the expression level of the *RNASE3* gene or of the expression level of *RNASE3*, in combination with the expression level of one or more of the genes *ALOX5*, *BAX*, *CCL5*, *CD40*, *CHI3L1*, *CPA3*, *IL1R2*, *IL4R*, *LGALS3*, *PI3*, *SELL* and *TGFB1*, for the prognosis of allergic asthma or for the differential diagnosis of mild allergic asthma and moderate allergic asthma.

In a preferred embodiment, the *in vitro* use or *in vitro* use of a kit further comprises the expression level of the *IL1R2* gene or of the expression level of the *IL1R2* gene in combination with the expression level of one or more of the genes *ALOX5*, *BAX*, *CCL5*, *CD40*, *CHI3L1*, *IL4R*, *LGLAS3*, *PI3*, *RNASE3* and *SELL*, for the prognosis of nonallergic asthma or for the differential diagnosis of mild or moderate nonallergic asthma and severe nonallergic asthma.

In a preferred embodiment, the *in vitro* use or *in vitro* use of a kit further comprises the expression level of the *RNASE3* gene or of the of the expression level of the *RNASE3* gene in combination with the expression level of one or more of the genes *ALOX5*, *BAX*, *CCL5*, *CD40*, *CHI3L1*, *CPA3*, *IL1R2*, *IL4R*, *LGLAS3*, *PI3*, *SELL* and *TGFB1*, for the prognosis of nonallergic asthma or for the differential diagnosis of mild nonallergic asthma and moderate nonallergic asthma.

The present invention also refers to:
1) an *in vitro* method for the diagnosis of nonallergic asthma, which comprises:
   a. assessing the expression level of a gene selected from *ALOX5*, *BAX*, *CCL5*, *CD40*, *CD86*, *CHI3L1*, *CPA3*, *IL10*, *IL1R2*, *IL4R*, *LGALS3*, *PHLDA1*, *PI3*, *PTGER2*, *RNASE3*, *SELL*, *SERPINB2* or *TGFB1* or any combination thereof comprising between one and eighteen genes in a biological sample obtained from the subject,
   b. wherein an increased expression level of the genes *ALOX5*, *BAX*, *CCL5*, *CD40*, *CD86*, *CHI3L1*, *CPA3*, *IL10*, *IL1R2*, *IL4R*, *LGALS3*, *PHLDA1*, *PI3*, *PTGER2*, *RNASE3*, *SELL*, *SERPINB2* or *TGFB1*, as compared to a pre-established reference value, is indicative that the subject does not suffer from nonallergic asthma,
   c. wherein a decreased expression level of the genes *ALOX5, BAX, CCL5, CD40, CD86, CHI3L1, CPA3, IL10, IL1R2, IL4R*, *LGALS3, PHLDA1*, *PI3, PTGER2, RNASE3, SELL, SERPINB2* or *TGFB1,* as compared to a pre-established reference value, is indicative that the subject suffers nonallergic asthma.
2) an *in vitro* method for the diagnosis of allergic asthma, which comprises:
   a. assessing the expression level of a gene selected from *ALOX5, BAX, CCL5*, *CD40*, *CD86*, *CPA3*, *IL10*, *IL1R2*, *IL4R*, *LGALS3*, *PHLDA1*, *PTGER2*, *RNASE3*, *SELL* or *TGFB1* or any combination thereof comprising between one and fifteen genes in a biological sample obtained from the subject,
   b. wherein an increased expression level of the genes *ALOX5*, *BAX*, *CCL5*, *CD40*, *CD86*, *CPA3*, *IL10*, *IL1R2*, *IL4R*, *LGALS3*, *PHLDA1*, *PTGER2*, *RNASE3*, *SELL* or *TGFB1*, as compared to a pre-established reference value, is indicative that the subject does not suffer from allergic asthma,
   c. wherein a decreased expression level of the genes *ALOX5*, *BAX*, *CCL5*, *CD40*, *CD86*, *CPA3*, *IL10*, *IL1R2*, *IL4R*, *LGALS3*, *PHLDA1*, *PTGER2*, *RNASE3*, *SELL* or *TGFB1*, as compared to a pre-established reference value, is indicative that the subject suffers from allergic asthma.
3) an *in vitro* method for the diagnosis of asthma, which comprises:
   a. assessing the expression level of a gene selected from *ALOX5*, *BAX*, *CCL5*, *CD40*, *CD86*, *CPA3*, *IL10*, *IL1R2*, *IL4R*, *LGALS3*, *PHLDA1*, *PTGER2*, *RNASE3*, *SELL* or *TGFB1* or any combination thereof comprising between one and fifteen genes in a biological sample obtained from the subject,
   b. wherein an increased expression level of the genes *ALOX5*, *BAX*, *CCL5*, *CD40*, *CD86*, *CPA3*, *IL10*, *IL1R2*, *IL4R*, *LGALS3*, *PHLDA1*, *PTGER2*, *RNASE3*, *SELL* or *TGFB1*, as compared to a pre-established reference value, is indicative that the subject does not suffer from asthma,
   c. wherein a decreased expression level of the genes *ALOX5*, *BAX*, *CCL5*, *CD40*, *CD86*, *CPA3*, *IL10*, *IL1R2*, *IL4R*, *LGALS3*, *PHLDA1*, *PTGER2*, *RNASE3, SELL* or *TGFB1,* as compared to a pre-established reference value, is indicative that the subject suffers from asthma.
4) an *in vitro* method for the diagnosis of allergic asthma or nonallergic asthma in a subject suffering from asthma, or for the differential diagnosis of allergic asthma and nonallergic asthma in a subject suffering from asthma, or for the prognosis of asthma in a subject suffering from asthma, which comprises:
   a. assessing the expression level of a gene selected from *ALOX5*, *BAX*, *CCL5*, *CD40*, *CHI3L1*, *CPA3*, *IL1R2*, *IL4R*, *LGALS3*, *PI3*, *RNASE3*, *SELL* or *TGFB1* or any combination thereof comprising between one and thirteen genes in a biological sample obtained from the subject,
   b. wherein an increased expression level of the *ALOXS*, *BAX*, *CCL5*, *CD40*, *CHI3L1*, *CPA3*, *IL1R2*, *IL4R*, *LGALS3*, *PI3*, *RNASE3*, *SELL* or *TGFB1*, as compared to a pre-established reference value, is indicative that the subject suffers from allergic asthma, and/or
   c. wherein a decreased expression level of the genes *ALOX5*, *BAX*, *CCL5*, *CD40*, *CHI3L1*, *CPA3*, *IL1R2*, *IL4R*, *LGALS3*, *PI3*, *RNASE3*, *SELL* or *TGFB1,* as compared to a pre-established reference value, is indicative that the subject suffers from nonallergic asthma.
5) an *in vitro* method for the prognosis of nonallergic asthma or for the differential diagnosis of mild nonallergic asthma and moderate nonallergic asthma, which comprises:
   a. assessing the expression level of a gene selected from *ALOX5*, *BAX*, *CCL5*, *CD40*, *CD86*, *CHI3L1*, *CPA3*, *IL1R2*, *IL4R*, *LGALS3*, *PI3*, *RNASE3, SELL* or *TGFB1* or any combination thereof comprising between one and fourteen genes in a biological sample obtained from the subject,
   b. wherein an increased expression level of the genes *BAX*, *CD40*, *LGALS3*, *PI3*, *RNASE3* or *SELL*, and/or a decreased expression level of the genes *ALOX5*, *CCL5*, *CHI3L1*, *CPA3*, *IL1R2*, *IL4R* or *TGFB1*, as compared to a pre-established reference value, is indicative that the subject suffers from mild nonallergic asthma, and/or
   c. wherein a decreased expression level of the genes *ALOX5*, *CCL5*, *CHI3L1*, *CPA3*, *IL1R2*, *IL4R* or *TGFB1*, and/or an increased expression level of the genes *BAX*, *CD40*, *LGALS3*, *PI3*, *RNASE3* or *SELL*, , as compared to a pre-established reference value, is indicative that the subject suffers from moderate nonallergic asthma.
6) an *in vitro* method for the prognosis of nonallergic asthma or for the differential diagnosis of mild nonallergic asthma and severe nonallergic asthma, which comprises:
   a. assessing the expression level of a gene selected from *ALOX5, BAX, CCL5, CD40, CHI3L1, IL1R2, IL4R*, *LGALS3, PI3, RNASE3, SELL* or *TGFB1* or any combination thereof comprising between one and twelve genes in a biological sample obtained from the subject,
   b. wherein an increased expression level of the genes *CCL5*, *CD40*, *LGALS3*, *SELL* or *TGFB1*, and/or a decreased expression level of the genes *ALOX5*, *BAX, CHI3L1, IL1R2, IL4R*, *PI3* or *RNASE3,* as compared to a pre-established reference value, is indicative that the subject suffers from mild nonallergic asthma, and/or
   c. wherein a decreased expression level of the genes *ALOX5*, *BAX*, *CHI3L1*, *IL1R2*, *IL4R*, *PI3* or *RNASE3*, and/or an increased expression level of the genes *CCL5*, *CD40*, *LGALS3*, *SELL* or *TGFB1*, as compared to a pre-established reference value, is indicative that the subject suffers from severe nonallergic asthma.
7) an *in vitro* method for the prognosis of nonallergic asthma or for the differential diagnosis of moderate nonallergic asthma and severe nonallergic asthma, which comprises:
   a. assessing the expression level of a gene selected from *ALOX5*, *BAX*, *CCL5*, *CD40*, *CHI3L1*, *IL1R2*, *IL4R*, *LGALS3*, *PI3*, *RNASE3*, *SELL* or *TGFB1* or any combination thereof comprising between one and twelve genes in a biological sample obtained from the subject,
   b. wherein an increased expression level of the genes *CCL5*, *CD40* or *SELL* or *TGFB1*, and/or a decreased expression level of the genes *ALOX5*, *BAX*, *CHI3L1*, *IL1R2*, *IL4R*, *LGALS3*, *PI3* or *RNASE3*, as compared to a pre-established reference value, is indicative that the subject suffers from moderate nonallergic asthma, and/or
   c. wherein a decreased expression level of the genes *ALOX5*, *BAX*, *CHI3L1*, *IL1R2*, *IL4R*, *LGALS3*, *PI3* or *RNASE3*, and/or an increased expression level of the genes *CCL5*, *CD40* or *SELL* or *TGFB1*, as compared to a pre-established reference value, is indicative that the subject suffers from severe nonallergic asthma.
8) an *in vitro* method for the prognosis of allergic asthma or for the differential diagnosis of mild allergic asthma and moderate allergic asthma, which comprises, which comprises:
   a. assessing the expression level of a gene selected from *ALOX5*, *BAX*, *CCL5*, *CD40*, *CHI3L1*, *CPA3*, *IL1R2*, *IL4R*, *LGALS3*, *PI3*, *RNASE3*, *SELL* or *TGFB1* or any combination thereof comprising between one and thirteen genes in a biological sample obtained from the subject,
   b. wherein an increased expression level of the genes *ALOX5*, *BAX*, *CCL5*, *CD40*, *CHI3L1*, *CPA3*, *IL1R2*, *IL4R*, *LGALS3*, *PI3*, *RNASE3*, *SELL* or *TGFB1,* as compared to a pre-established reference value, is indicative that the subject suffers from mild allergic asthma, and/or
   c. wherein a decreased expression level of the genes *ALOX5*, *BAX*, *CCL5*, *CD40*, *CHI3L1*, *CPA3*, *IL1R2*, *IL4R*, *LGALS3*, *PI3*, *RNASE3*, *SELL* or *TGFB1,* as compared to a pre-established reference value, is indicative that the subject suffers from moderate allergic asthma.
9) an *in vitro* method for the prognosis of allergic asthma or for the differential diagnosis of mild allergic asthma and severe allergic asthma, which comprises:
   a. assessing the expression level of a gene selected from *ALOX5*, *BAX*, *CCL5*, *CD40*, *CHI3L1*, *CPA3*, *IL1R2*, *IL4R*, *LGALS3*, *PI3*, *RNASE3*, *SELL* or *TGFB1* or any combination thereof comprising between one and thirteen genes in a biological sample obtained from the subject,
   b. wherein an increased expression level of the genes *ALOX5*, *BAX*, *CCL5*, *CD40*, *CPA3*, *IL1R2*, *IL4R*, *LGALS3*, *RNASE3*, *SELL* or *TGFB1*, and/or a decreased expression level of the genes *CHI3L1* or *PI3,* as compared to a pre-established reference value, is indicative that the subject suffers from mild allergic asthma, and/or
   c. wherein a decreased expression level of the genes *CHI3L1* or *PI3*, and/or an increased expression level of the genes *ALOX5*, *BAX*, *CCL5*, *CD40*, *CPA3*, *IL1R2*, *IL4R*, *LGALS3*, *RNASE3*, *SELL* or *TGFB1*, as compared to a pre-established reference value, is indicative that the subject suffers from severe allergic asthma.
10) an *in vitro* method for the prognosis of allergic asthma or for the differential diagnosis of moderate allergic asthma and severe allergic asthma, which further comprises:
   a. assessing the expression level of a gene selected *ALOXS*, *BAX*, *CCL5*, *CD40*, *CHI3L1*, *CPA3*, *IL1R2*, *IL4R*, *LGALS3*, *PI3*, *RNASE3*, *SELL* or *TGFB1* or any combination thereof comprising between one and thirteen genes in a biological sample obtained from the subject,
   b. wherein an increased expression level of the genes *ALOX5*, *BAX*, *CCL5*, *CD40*, *CPA3*, *IL4R*, *LGALS3*, *PI3*, *RNASE3*, *SELL* or *TGFB1*, and/or a decreased expression level of the genes *CHI3L1*, *IL1R2* or *PI3*, as compared to a pre-established reference value, is indicative that the subject suffers from moderate allergic asthma, and/or
   c. wherein a decreased expression level of the genes *CHI3L1*, *IL1R2* or *PI3*, and/or an increased expression level of the genes *ALOX5*, *BAX*, *CCL5*, *CD40*, *CPA3*, *IL4R*, *LGALS3*, *PI3*, *RNASE3*, *SELL* or *TGFB1*, as compared to a pre-established reference value, is indicative that the subject suffers from severe allergic asthma.

In preferred embodiment the subject is a mammal.

In preferred embodiment the *in vitro* the subject is a human.

In preferred embodiment the biological sample is blood, serum or plasma.

The present invention also refers to:
11) the *in vitro* use of the genes or combinations of genes mentioned in the methods 1) to 10) above for use in the applications described in those methods.
12) the *in vitro* use of a kit comprising reagents for the assessment of the expression level of the genes mentioned in the methods 1) to 10) above for use in the applications described in those methods.

Finally, the invention also refers to an *in vitro* method for detecting and/identifying biomarkers signatures for the diagnosis of asthma, or for the diagnosis of allergic asthma or nonallergic asthma in a subject suffering from asthma, or for the differential diagnosis of allergic asthma and nonallergic asthma in a subject suffering from asthma, or for the prognosis of asthma in a subject suffering from asthma, which comprises:
a) assessing the expression level of at least one gene selected from *ALOX5*, *BAX*, *CCL5*, *CD40*, *CD86*, *CHI3L1*, *CPA3*, *IL10*, *IL1R2*, *IL4R*, *LGALS3*, *PHLDA1*, *PI3*, *PTGER2*, *RNASE3*, *SELL*, *SERPINB2* or *TGFB1*, in a biological sample obtained from the subj ect,
b) wherein a deviation of the expression level of the genes of step a), in a subset of subjects as compared to a pre-established reference value measured in another subset of subjects, is an indication that the biomarker signature may be used for the diagnosis of asthma, or for the diagnosis of allergic asthma or nonallergic asthma in a subject suffering from asthma, or for the differential diagnosis of allergic asthma and nonallergic asthma in a subject suffering from asthma.

In preferred embodiment the subject is a mammal.

In preferred embodiment the *in vitro* the subject is a human.

In preferred embodiment the biological sample is blood, serum or plasma.

In a preferred embodiment, the present invention is a computer-implemented invention, wherein a processing unit (hardware) and a software are configured to: a) Receive the expression level of the above-mentioned genes, b) process the expression level values for finding substantial variations or deviations, and c) provide an output through a terminal display of the variation or deviation of the expression level, wherein the variation or deviation of the expression level is indicative of whether the subject is suffering from allergic asthma or nonallergic asthma.

It is important to mention that while the above-mentioned embodiments are directed towards detecting the level of expression of the gene, it would also be possible to use the level of expression of the protein instead of the gene for purposes mentioned in each of the embodiments. The reason behind this is that the level of expression of a gene generally correlates with the level of expression of the protein encoded by the gene.

All the methods described above could be potentially implemented through an algorithm.

In the context of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- The term "consisting of" means including, and it is limited to, whatever follows the phrase "consisting of'. Thus, the phrase "consisting of" indicates that the listed elements are required or mandatory, and that no other elements may be present.
- The term "asthma" means a chronic inflammatory disease of the respiratory tract, in the pathogenesis of which various cells and mediators of inflammation intervene, conditioned in part by genetic factors and which presents with bronchial hyperresponsiveness (HRB) and a variable airflow obstruction, totally or partially reversible, either by the action of medications or spontaneously.
- The term "nonallergic asthma" is understood as the clinical diagnosis of asthma made by a specialist Allergist or Pulmonologist, according to the criteria of the Spanish Guide to the Management of Asthma (GEMA) and without any associated allergic symptoms (clinical history). not suspected of any allergy and negative skin tests against a battery of common allergens).
- The term "allergic asthma" means the clinical diagnosis of asthma made by a specialist Allergist or Pulmonologist, according to the criteria of the Spanish Asthma Management Guide (GEMA), associated with characterized allergic symptoms (history of suspicion of allergic diseases, positive skin tests against any allergen from a battery of common allergens, elevated total IgE).
- The terms "mild allergic asthma" or "mild nonallergic asthma" refers to the clinical diagnosis of asthma severity made by a specialist Allergist or Pulmonologist, according to the criteria of the Spanish Asthma Management Guide (GEMA), associated with symptoms severity **(Table 2),** specifically mild asthma means more than 2 daytimes symptoms a week; need to relief medication more than 2 times a week (but not daily); nightly symptoms more than 2 times a month, some activity limitation, Pulmonary function <80% and one or none exacerbations a year.
- The terms "moderate allergic asthma" or "moderate nonallergic asthma" refers to the clinical diagnosis of asthma severity made by a specialist Allergist or Pulmonologist, according to the criteria of the Spanish Asthma Management Guide (GEMA), associated with symptoms severity **(Table 2),** specifically moderate asthma means daily symptoms; need to relief medication daily; nightly symptoms more than 1 times a week, quite activity limitation, Pulmonary function >60%-<80% and two or more exacerbations a year.
- The terms "severe allergic asthma" or "severe nonallergic asthma" refers to the clinical diagnosis of asthma severity made by a specialist Allergist or Pulmonologist, according to the criteria of the Spanish Asthma Management Guide (GEMA), associated with symptoms severity **(Table 2),** specifically severe asthma means continuous symptoms; need to relief medication several times a day; frequents nightly symptoms, much activity limitation, Pulmonary function <60% and two or more exacerbations a year.
- The term "pre-established reference value" refers to a value that is determined in subjects that belong to a reference group. This value enables the discrimination between case and control subjects. To clarify the reference group used in each case we provide the following examples:
   ∘ If the method is intended for the diagnosis of condition X, and an increased/decreased expression of a genes A, B and C is indicative that the subject suffers from condition X, then the pre-established reference value refers to a value determined in subjects that do not suffer from condition X, or by comparing subjects that suffer from condition X with subjects that do not suffer from condition X.
   ∘ If the method is intended for the differential diagnosis of condition X and condition Y, and an increased/decreased expression of a genes A, B and C is indicative that the subject suffers from condition X, then the pre-established reference value refers to a value determined in subjects that suffer from condition Y, or by comparing subjects that suffer from condition Y with subjects that suffer from condition X.
   ∘ If the method is intended for the differential diagnosis of condition X and condition Y, and an increased/decreased expression of a genes A, B and C is indicative that the subject suffers from condition Y, then the pre-established reference value refers to a value determined in subjects that suffer from condition X, or by comparing subjects that suffer from condition X with subjects that suffer from condition Y.
The "pre-established threshold value" can be determined experimentally, empirically or theoretically. A "threshold value" can also be arbitrarily selected based upon the existing experimental and/or clinical conditions, as would be recognized by a person skilled in the art. The "threshold value" has to be determined in order to obtain the optimal sensitivity and specificity according to the function of the test and the benefit/risk balance (clinical consequences of false positive and false negative). Typically, the optimal sensitivity and specificity (and so the "threshold value") can be determined using a Receiver Operating Characteristic (ROC) curve based on experimental data.

### Description of the figures

**Figure 1****.** Response of the epithelium to external elements and subsequent activation of the immune response in non-eosinophilic (left) and eosinophilic (right) asthma. The activation of the epithelium in response to viruses, tobacco, allergens or other elements triggers a release of cytokines that activate innate lymphoid cells (ILC) and dendritic cells (DC), the latter being involved in the differentiation of Th0 lymphocytes into Th1, Th2 or Th17. These differentiated lymphocytes, in turn, secrete cytokines that activate neutrophils recruited to the airways (Th1 and Th17) or eosinophils (Th2). Furthermore, Th2 lymphocytes promote the differentiation of B lymphocytes into IgE-producing plasma cells, which bind to mast cells, allowing their allergen-dependent activation. These activated cells secrete different proinflammatory molecules that produce epithelial damage, increased mucus production, angiogenesis and permeability of blood vessels, hypertrophy and hyperresponse of smooth muscle, etc.; that cause the characteristic symptoms of asthma.

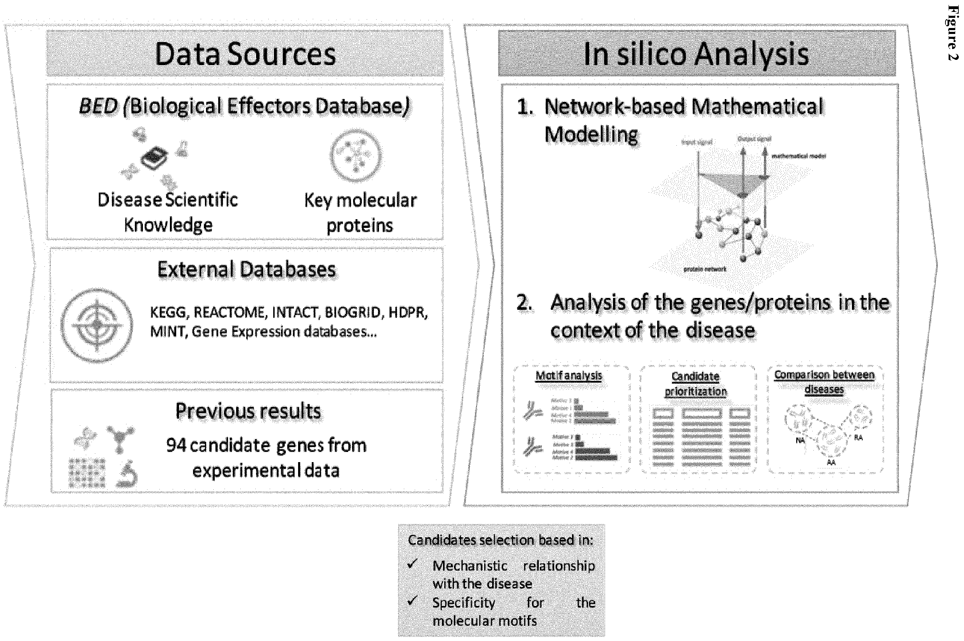

**Figure 2****.** Scheme of the process followed for the systems biology analysis of the previously identified genes. Information is taken from different data sources, including experimental results, and network-based mathematical models are developed for the diseases studied, through which the genes and proteins of interest are analyzed. Thus, the best candidates are selected based on their specificity, triggering capacity and mechanistic involvement with the disease.

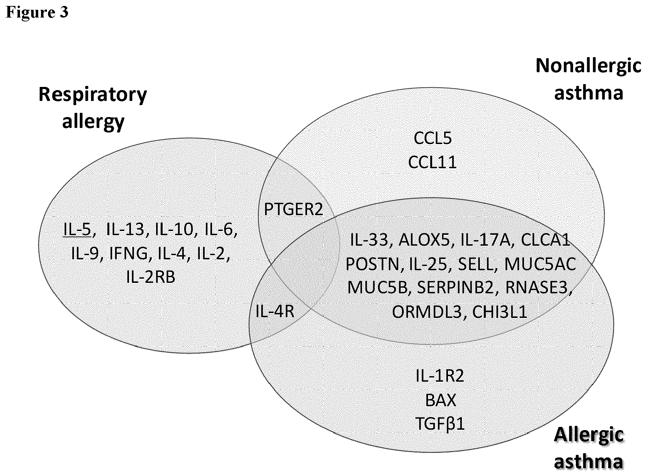

**Figure 3****.** Venn diagram that shows the proteins with a high or very high association (underlined) with the diseases studied. It is observed that some of these proteins are specific to one of the diseases, while others are common to two of them. None of the proteins analyzed had a high association with the three diseases.
**Figure 4****.** Algorithm proposed for the diagnosis of asthma, based on the experimental results. Following this diagram, the asthma diagnosis could be performed based only in the measurement of the expression of one gene: *CPA3.* Also, the expression of two more genes, *LGALS3* and *TGFB1*, could discriminate allergic asthma and nonallergic asthmatic patients.

With three more genes, *RNASE3*, *IL4R* and *IL1R2*, the severity discrimination could be performed.

### Detailed description of the invention

The present invention is illustrated by means of the Examples set below without the intention of limiting its scope of protection.

### Example 1. Material and Methods

### Example 1.1. Study Design: Subjects

The study population comprised 93 unrelated subjects, 24 healthy control (HC) subjects, 18 patients with nonallergic asthma (NA) and 51 patients with allergic asthma (AA), who followed the selection criteria: aged 18-75 years, with no symptoms of respiratory diseases for the HC subjects or diagnosed with asthma at least 1 year before inclusion in the study. Demographic and clinical characteristics are summarized in **Table 5.** For each patient, two longitudinal samples separated by two years were obtained from the biobank of the cohort from the MEGA project (T0 and T2), except for one AA patient where the follow-up was not possible. Patients were diagnosed and recruited in the allergy and pulmonology departments of the *Fundación Jiménez Diaz* Hospital in Madrid, the allergy department of *La Paz* Hospital in Madrid, and *Complejo Hospitalario de Navarra* in Pamplona. Samples were processed in each hospital. For the control group, the subjects were recruited in the allergy departments of *Fundación Jiménez Diaz* Hospital in Madrid, Policlinic Hospital in Seville, and *San Cecilio* University Hospital in Granada, Spain; and samples were processed in the immunology department of the Health Research Institute-*Fundación Jiménez Díaz-UAM* (IIS-FJD-UAM) in Madrid.

For all subjects, tests of pulmonary function were carried out by determining the predicted percentage of forced vital capacity (%FVC) and the forced expiratory volume in 1 s (%FEV1) at the moment the two samples were obtained. NA patients were diagnosed with asthma disease but had a negative skin prick test while AA patients were asthmatic and allergic to at least one airborne allergen. In both groups, the asthma severity was diagnosed as severe, moderate or mild according to the Spanish Guidelines for the Management of Asthma (GEMA). For all the asthmatic patients, at the moment of inclusion in the study (T0) a deep diagnosis was performed, analysing inflammatory markers such as blood and sputum eosinophils and neutrophils, as well as the FeNO. The HC group was made up of healthy subjects with no history of respiratory diseases or allergic symptoms, and were negative to skin prick test against a panel of common allergens, including mites (*Dermatophagoides pteronyssinus*, *Dermatophagoides farinae*, and *Lepidoglyphus destructor*), epithelia (cat and dog), cockroaches (*Blatella orientalis* and *Blatella germanica*), pollens (*Cypress*, *banana shadow*, olive, mixture of grasses, *Artemisia*, *Parietaria*, and *Salsola*), and fungi (*Alternaria*, *Cladosporium*, *Aspergillus*, and *Penicillium*).

Written informed consent was obtained from each subject in accordance with the Declaration of Helsinki. Ethical approval for the study was obtained from the research ethics committee of the IIS-FJD-UAM.

**Table 5. Demographic and clinical characteristics of the study population.**

| | | | **Healthy control subjects (HC)** | **Nonallergic asthmatic patients (NA)** | **Allergic asthmatic patients (AA)** |
|---|---|---|---|---|---|
| **N** | | | 24 | 18 | 51 |
| **Age (years), mean ± SD** | | | 37.46 ± 11.5 | 52.89 ± 11.2# | 46.88 ± 12.8* |

| **Gender, N (%)** | | | | | |
|---|---|---|---|---|---|
| | **Male** | | 9 (37.5) | 7 (38.9) | 15 (29.4) |
| | **Female** | | 15 (62.5) | 11 (61.1) | 36 (70.6) |

| **Smoking Habit, N (%)** | | | | | |
|---|---|---|---|---|---|
| | **Smokers** | | 2 (8.3) | 2(11.1) | 5 (9.8) |
| | **Ex-Smokers** | | 0(0) | 9 (50) | 15 (29.4) |
| | **Non-Smokers** | | 5 (20.8) | 6 (33.3) | 25 (49) |
| | **No data** | | 17 (70.8) | 1 (5.6) | 6 (11.8) |

| **Asthma severity, N (%)** | | | | | |
|---|---|---|---|---|---|
| | **Severe** | | - | 4 (22.2) | 18 (35.3) |
| | **Moderate** | | - | 8 (44.5) | 15 (29.4) |
| | **Mild** | | - | 6 (33.3) | 18 (35.3) |

| **Spirometry, mean ± SD** | | | | | |
|---|---|---|---|---|---|
| | **FEV₁ (%)** | **T0** | 99.84 ± 7.6 | 98.09 ± 21.7 | 87.71 ± 20 |
| | | **T2** | - | 93.81 ± 16.2 | 83.76 ± 18.9* |
| | **FVC (%)** | **T0** | 103.95 ± 6.9 | 110.71 ± 20.2 | 98.19 ± 18.3† |
| | | **T2** | - | 105.98 ± 14.4 | 97.61 ± 15.4† |
| | **FEV₁/FVC** | **T0** | 82.53 ± 2.2 | 83.05 ± 15 | 77.27 ± 10.2 |
| | | **T2** | - | 87.06 ± 12.9 | 74.95 ± 11.4† |
| **FeNO (ppb), mean ± SD** | | | 11.1 ± 5.9 | 31.64 ± 26.1 | 41.49 ±34.1 |
| **Total IgE (kU/l), mean ± SD** | | 53.65 ± 80.7 | 140.02 ± 170* | 414.3 ± 467.4#, †† | |

| **Blood analysis** | | | | | |
|---|---|---|---|---|---|
| | **Eosinophils (cells/mm³), mean ± SD** | 250 ± 171.6 | 423.44 ± 199.4* | 334.04 ± 231.5† | |
| | **Patients with ≥300 eosinophils/mm³, N (%)** | 5 (20.8) | 13 (72.2) | 29 (56.9) | |
| | **Neutrophils (cells/mm3), mean ± SD** | - | 4227.69 ± 1355.5 | 3747.94 ± 1509.9 | |

| **Sputum analysis** | | | | | |
|---|---|---|---|---|---|
| | **Eosinophils (%), mean ± SD** | - | 16 ± 27.9 | 8.76 ± 16.3 | |
| | **Patients with ≥3% eosinophils, N (%)** | - | 5 (27.8) | 12 (23.5) | |
| | **Neutrophils (%), mean ± SD** | - | 40 ± 30.4 | 32.29 ± 25.4 | |

| **Cellular profile, N (%)** | | | | | |
|---|---|---|---|---|---|
| | **Eosinophilic** | | 4 (22.2) | 10 (19.6) | |
| | **Mixed** | | 1 (5.6) | 2 (3.9) | |
| | **Neutrophilic** | | 4 (22.2) | 6 (11.8) | |
| | **Paucigranulocytic** | | 3 (16.7) | 11 (21.6) | |
| | **No data** | | 6 (33.3) | 22 (43.1) | |

| | | | | | |
|---|---|---|---|---|---|
| T0: results at the time the patients enter the study, T2: results 2 years after. %FVC: percentage of forced vital capacity; %FEV1: percentage of forced expiratory volume in 1 s, FeNO: fractional exhaled nitric oxide. *, **, ^{#}: differences vs HC group (p<0.05, p<0.005, p<0.001, respectively). ^{†}, ^{††}: differences vs NA group (p<0.05, p<0.01, respectively). | | | | | |

### Example 1.2. Isolation of peripheral blood mononuclear cells (PBMCs)

Peripheral blood samples were obtained from all the subjects in the moment they were included in the study (T0) and, in the case of asthmatic patients, two years later (T2). PBMCs were isolated from heparinized blood by gradient centrifugation using Lymphoprep (Comercial Rafer, Zaragoza, Spain) following the manufacturer's instructions. Cell isolation was performed under sterile conditions using endotoxin-free reagents.

### Example 1.3. RNA extraction

RNA was obtained from PBMCs (10⁶ cells) using the Trizol method (Invitrogen, Carlsbad, CA, USA). Quantification and purity of RNA was checked by spectrophotometry using a spectrophotometer (Nanodrop ND-1000, Bonsái Technologies Group, Madrid, Spain).

### Example 1.4. Gene selection

### Prioritization of asthma biomarkers by systems biology

The first step of the study was the systems biology analysis of the group's previous experimental results, where the expression of 94 genes was analyzed in peripheral samples (PBMC) from healthy subjects, patients with respiratory allergy, allergic asthmatic patients and nonallergic asthmatic patients. allergic. The selection of these 94 genes was made based on their relationship with asthma according to an exhaustive literature search and previous studies by our group. Following the scheme shown in **Figure 2****,** the best candidates were prioritized and selected based on: their specificity for one or more of the pathologies studied (candidate biomarkers), probability of acting as triggers and mechanistic relationship with the disease (therapeutic targets).

### Definition of molecular biomarkers of asthma in peripheral samples

The systems biology analysis allowed us to define a list of genes with a high specificity for one or two of the diseases analyzed: respiratory allergy, allergic asthma and nonallergic asthma. These genes are summarized in **Figure 3****.**

26 genes were selected according to previous results of our group, based on their theoretical specificity and relationship with asthma and their ability of discriminate between asthmatic patients and healthy controls, as well as between asthma severities **(Table 2).**

**Table 6. Genes studied, chromosome location and primers used for the gene expression analysis.**

| **Gene** | **Chromosome location (GRCh38/hg38)** | **Primer reference (Thermo Fisher Scientific)** |
|---|---|---|
| *ALOX5* | Chr.10: 45374166 - 45446121 | Hs01095330_m1 |
| *BAX* | Chr.19: 48954825 - 48961798 | Hs00180269_m1 |
| *CCL11* | Chr.17: 34285668 - 34288180 | Hs00237013_m1 |
| *CCL17* | Chr.16: 57396076 - 57416063 | Hs00171074_m1 |
| *CCL5* | Chr.17: 35871491 - 35880373 | Hs00982282_m1 |
| *CD40* | Chr.20: 46118242 - 46129745 | Hs01002913_g1 |
| *CD86* | Chr.3: 122055362 - 122121143 | Hs01567026_m1 |
| *CHI3L1* | Chr.1: 203178931 - 203186794 | Hs01072228_m1 |
| *CLCA1* | Chr.1: 86468843 - 86500294 | Hs00154490_m1 |
| *CPA3* | Chr.3: 148865256 - 148897091 | Hs00157019_m1 |
| *CXCL8 (IL8)* | Chr.4: 73740506 - 73743716 | Hs00174103_m1 |
| *IL10* | Chr.1: 206767603 - 206772494 | Hs00961622_m1 |
| *IL17A* | Chr.6: 52186387 - 52190638 | Hs00174383_m1 |
| *IL1R2* | Chr.2: 101991805 - 102028544 | Hs01030384_m1 |
| *IL4R* | Chr.16: 27313668 - 27364778 | Hs00166237_m1 |
| *LGALS3* | Chr.14: 55129217 - 55145430 | Hs00173587_m1 |
| *MSR1* | Chr.8: 16107878 - 16192791 | Hs00234007_m1 |
| *MUCSB* | Chr.11: 1223065 - 1262176 | Hs00861595_m1 |
| *PHLDA1* | Chr.12: 76025447 - 76031776 | Hs00705810_s1 |
| *PI3* | Chr.20: 45174899 - 45176544 | Hs00160066_m1 |
| *POSTN* | Chr.13: 37562582 - 37598844 | Hs01566750_m1 |
| *PTGER2* | Chr.14: 52314298 - 52328606 | Hs04183523_m1 |
| *RNASE3* | Chr.14: 20891403 - 20892348 | Hs01923184_s1 |
| *SELL* | Chr.1: 169690665 - 169711702 | Hs00174151_m1 |
| *SERPINB2* | Chr.18: 63887705 - 63903890 | Hs01010736_m1 |
| *TGFB1* | Chr.19: 41330531 - 41353933 | Hs00998133_m1 |

### Example 1.5. Differential gene expression analysis by RT-qPCR

Gene expression was analysed through real time semi quantitative PCR. Briefly, from three to eight hundred nanograms of RNA from each subject were converted to cDNA trough reverse transcription, using "High-capacity RNA to cDNA kit" (Applied Biosystems, Foster City, CA, USA). Then, quantitative real-time PCR was performed using the TaqMan Gene Expression System (Applied Biosystems), with the predesigned primers specified in **Table 1.** For *MSR1*, *CHI3L1* and *PI3,* the amplification was performed with 96-well plates in a 7500 Real-Time PCR System (Applied Biosystems). The rest of the genes were amplificated using 384-well microfluidic cards with the HT7900 System (Applied Biosystems) in the Scientific Park of Cantoblanco (Madrid, Spain). The gene expression of the selected genes was analysed in duplicates or triplicates, and expression of *18S* gene was used as a reference.

Finally, Relative Quantification (RQ) in each clinical group respecting control group was calculated according to the cycle threshold (Ct) method, with the gene expression expressed as 2^{-ΔΔCt}, where ΔΔCt = (ΔCt_{clinical group}) - (ΔCt_{control group}) and ΔCt = (Ct_{study gene}) - (Ct_{18S}).

### Example 1.6. Statistical analysis

Multiple comparisons were performed, using the R program. First, differential gene expression between groups was assessed using the Non-parametric Mann-Whitney test. Statistical significance was established as an adjusted two-tailed P value <0.05 and, in the gene expression analysis, an RQ<0.5. Also, expression stability through time was assessed by the analysis of the correlation between the expression at both times, using the Nonparametric Spearman test. A strong correlation was considered when the r coefficient value was ≥0.80, moderate when it was between the values 0.79-0.50, and weak when r<0.50.

### Example 1.7. ROC Curve analysis

Sensitivity and specificity of each biomarker to discriminate between clinical groups and severities were assessed through ROC curve graphs, performed using R program. According to the Area Under the Curve (AUC) obtained for each comparison, biomarkers were classified in: poor test (AUC=0.50-0.60), regular test (AUC=0.61-0.75), good test (AUC=0.76-0.90), very good test (AUC=0.91-0.97), and excellent test (AUC=0.98-1). Only the results with AUC values between 0.70 and 1 and CI (confidence interval) of a 95% were considered statistically relevant.

### Example 2. Results

### Example 2.1. Gene expression results in the clinical groups and healthy subjects

We analyzed the 26 genes in all groups and compared their expression in asthma groups respecting HC **(Table 7).** We were not able to detect *CCL11*, *CCL17*, *CLCA1*, *IL17A*, *MUC5B* and *POSTN* in any sample, thus only results of the other 20 genes are shown.

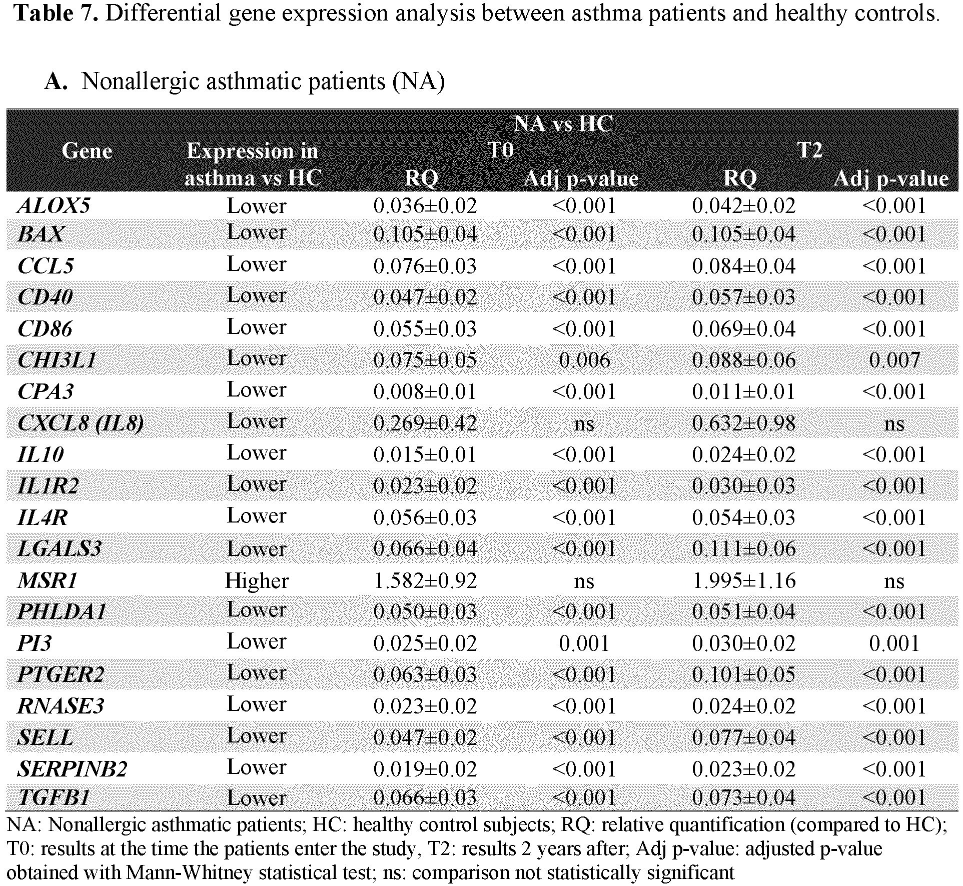

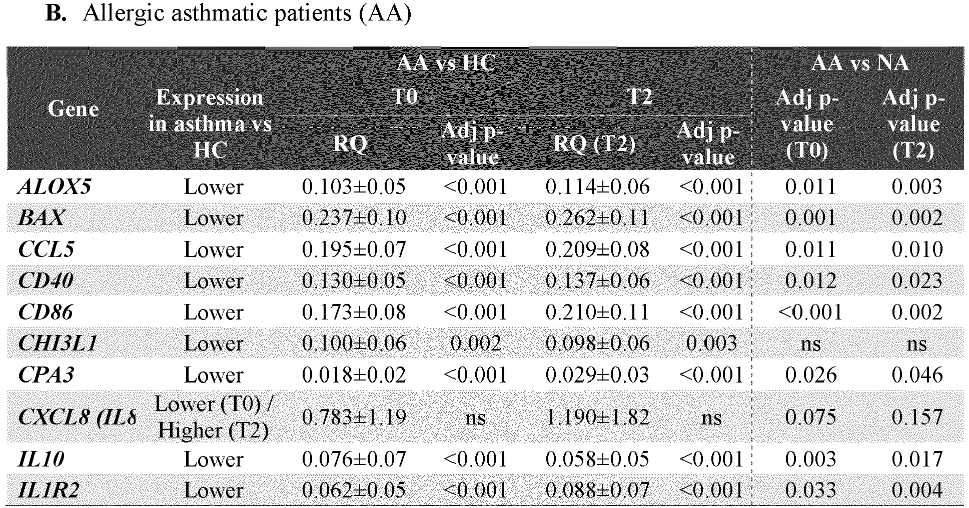

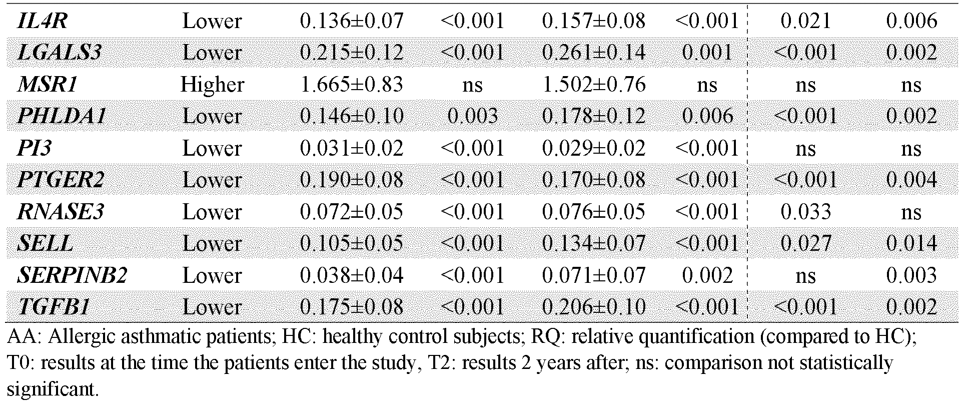

As it is shown, the only gene with a higher gene expression in the clinical groups than in the healthy subjects was *MSR1*, but without any statistically significant difference. From the other genes, statistically significant differences were observed between both clinical groups and HC in all of them, except in *IL-8*, and at both moments analysed (T0 and T2). Also, in these genes the decrease in the expression was more pronounced in the NA group than in the AA group, with statistically significant differences between both clinical groups observed all genes except *CHI3L1*, *IL-8*, *MSR1* and *PI3.* In the case of *RNASE3*, the statistically significant differences between NA and AA were observed only in T2, while in *SERPINB2* in T0. In the rest of the genes the significant differences were observed in both times. Giving the observed results, no deeper analysis was performed for *MSR1* or *IL-B.*

Interestingly, when the results obtained in the patients were analysed according to their asthma severity **(Table 8),** a similar gene expression levels are observed in the NA subgroups, independently of their severity **(Table 8A),** but in the AA subgroups the gene levels are inversely proportional to the asthma severity, with the highest gene expression in the mild patients **(Table 8B),** in which expression levels were closer to the observed in HC. Also, statistically significant differences with HC group are observed in all the severity subgroups in NA in almost all genes; while in the AA subgroups statistically significant differences with HC are only observed in the severe and moderate but not in the mild patients, with the exception of *CHI3L1* and *PI3.* This suggests a different behavior of the expression of these genes according to severity in both asthma groups. Additionally, statistically significant differences between both asthma groups are hardly observed in severe or moderate patients, but in almost all genes in the mild patients (data not shown).

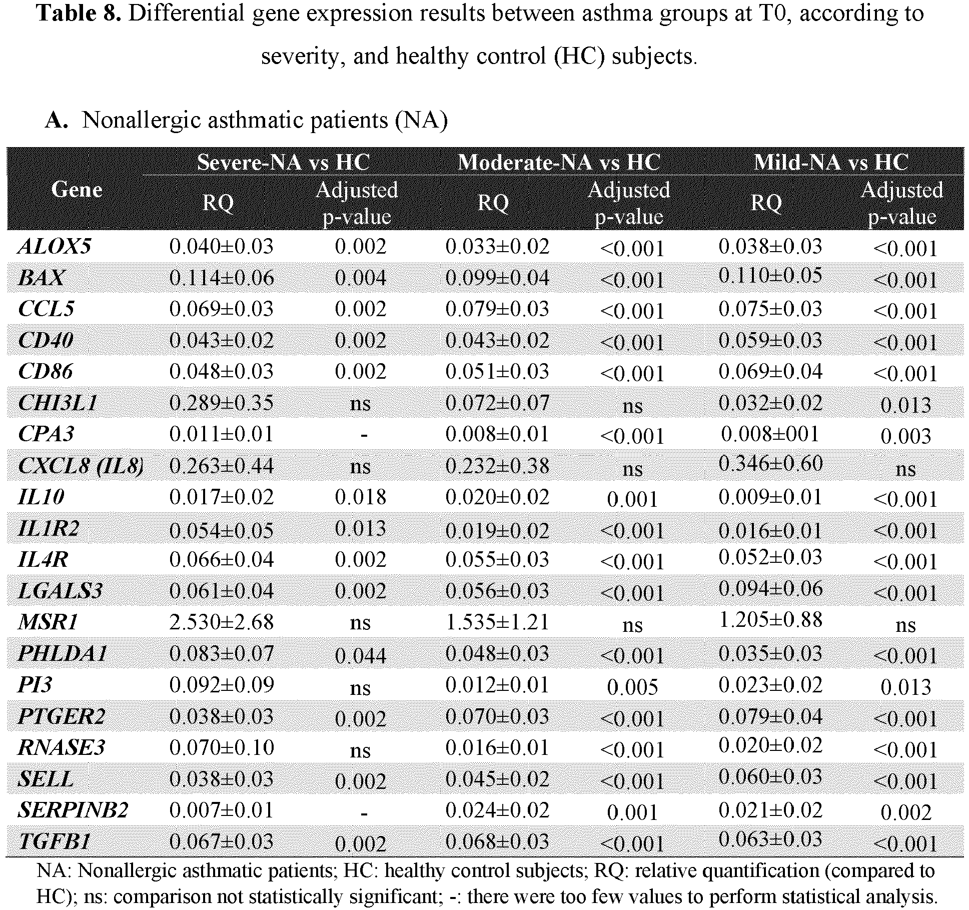

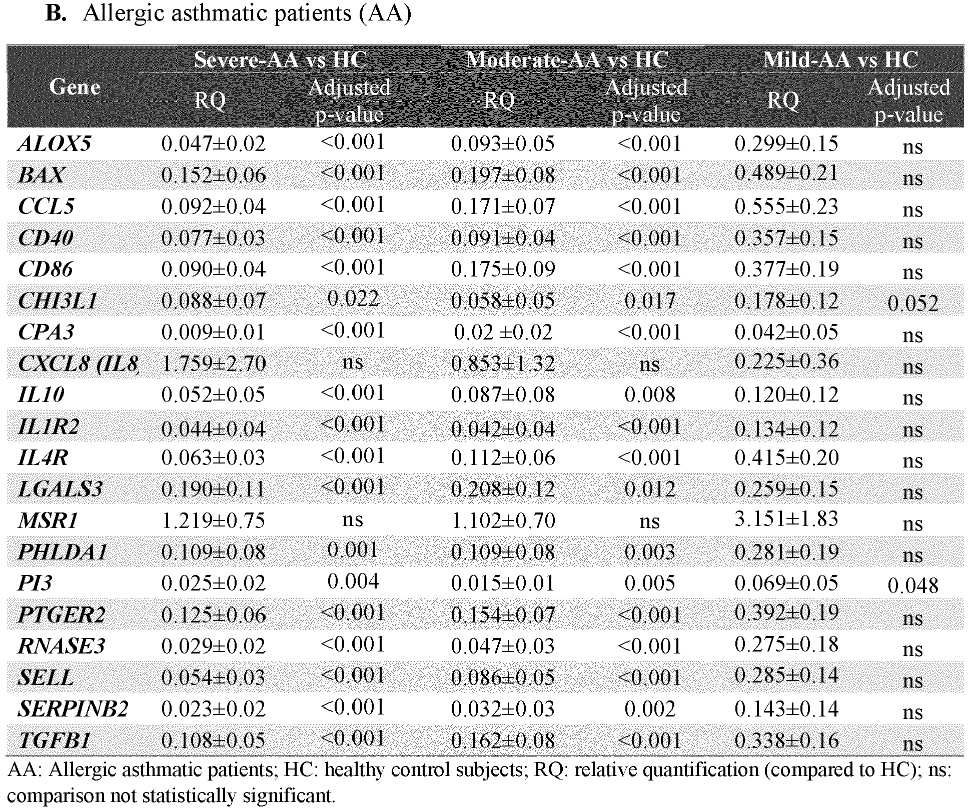

### Example 2.2. Stability of the gene expression along a two year longitudinal study

A desirable characteristic of a good biomarker is its stability through time, as long as the disease stay stable, making easier the determination of the threshold levels. Most of the asthmatic patients in this work maintained their disease controlled and stable through the two years analyzed, so stability of the 16 selected genes was assessed with a correlation analysis. These results are shown in the **Table 9.**

A statistically significant correlation between the expression at T0 and at T2 in both groups is observed in *ALOX5*, *BAX*, *CCL5*, *IL4R*, *TGFB1*, *CHI3L1* and *PI3.* Also, CD40, *CPA3*, *RNASE3* and *SELL* also showed a good correlation in the AA group, but not in the NA; while *IL1R2* and *LGALS3* showed a good correlation in NA, but not AA.

The rest of the genes (*CD86*, *IL10*, *PHLDA1*, *PTGER2* and *SERPINB2*) did not show a statistically significant correlation between both moments analised, in any group, showing a worst stability in time. However, those genes showed significant differences between clinical phenotypes at T0 and T2, meaning they could be potential asthma biomarkers. For this, with the aim to simplify the interpretation of data and facilitate the establishment of a single threshold for the discrimination of asthma (useful at any moment the patient is diagnosed), no further analysis was performed with those genes.

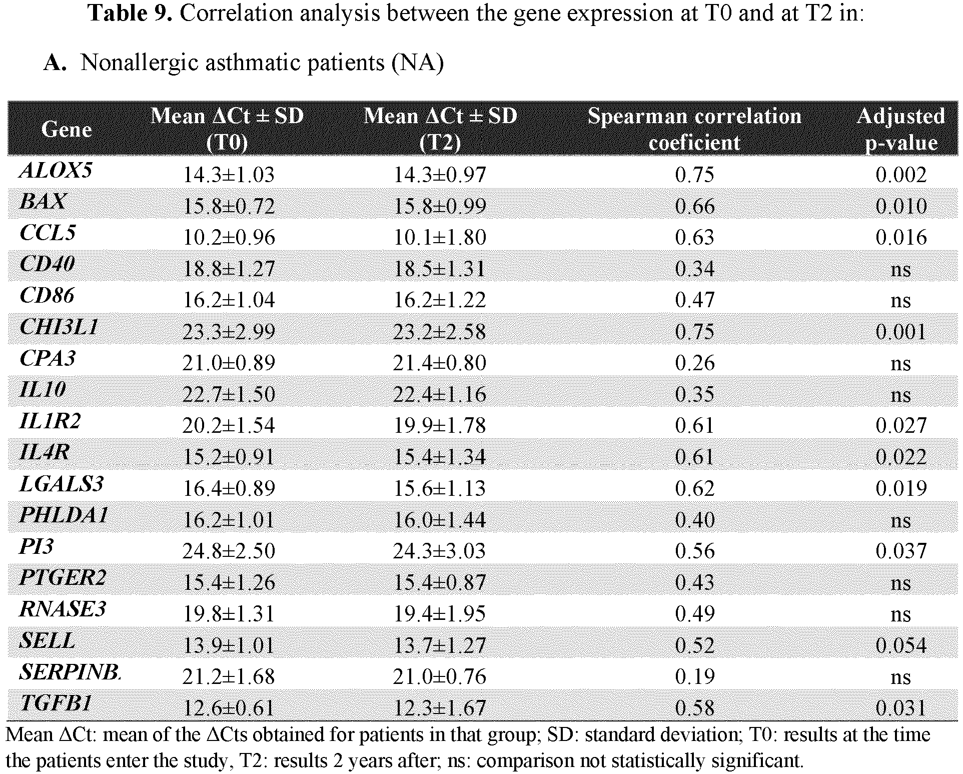

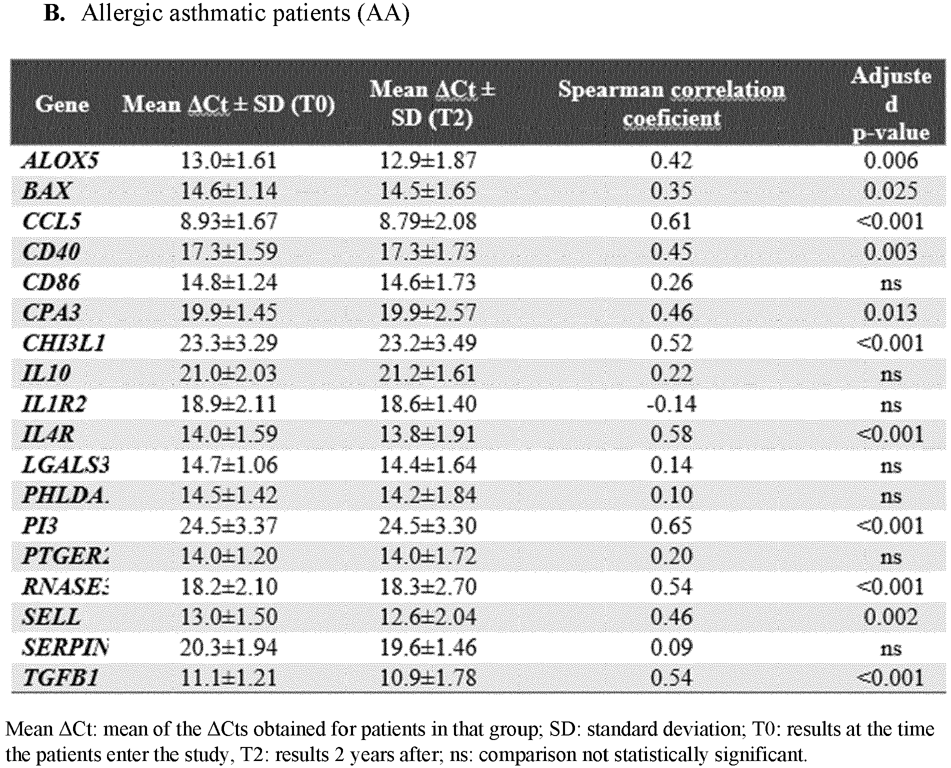

### Example 2.3. Potential of the studied genes to discriminate between phenotypes

The previous analyses allowed us to select 13 genes, that were differentially expressed and stable in at least one of the clinical conditions, for further analyses: *ALOX5*, *BAX*, *CCL5*, *CD40*, *CHI3L1*, *CPA3*, *IL1R2*, *IL4R*, *LGALS3*, *PI3*, *RNASE3*, *SELL* and *TGFB1.* Our next step was to identify the ones with the highest potential for the discrimination of the three clinical conditions: NA, AA and HC. For that, sensitivity and specificity for each discrimination were assessed through ROC curve analyses, using the data obtained at T0, and the genes were then classified according to their ability to separate groups based on the AUC value obtained for each comparison. The results are shown in the **Table 10.**

For the discrimination between the healthy subjects and the NA patients **(Table 10A),** the genes with the best AUC values were *ALOX5*, *CCL5*, *CD40*, *CPA3*, *IL4R*, *LGALS3*, *SELL* and *TGFB1*, classified as excellent tests. However, the other five genes also showed high AUC values. On the other hand, for the comparison between AA patients and HC subjects **(Table 10B),** no gene were classified as excellent test, being *CPA3* the gene with the highest AUC, classified as very good test. The rest of the genes showed a good capacity to discriminate these two groups. Finally, the AUC values obtained for the discrimination of NA and AA (Table 10C) were lower than the observed in the other comparisons, being *BAX*, *LGALS3* and *TGFB1* the ones with the highest AUC, classified as good tests.

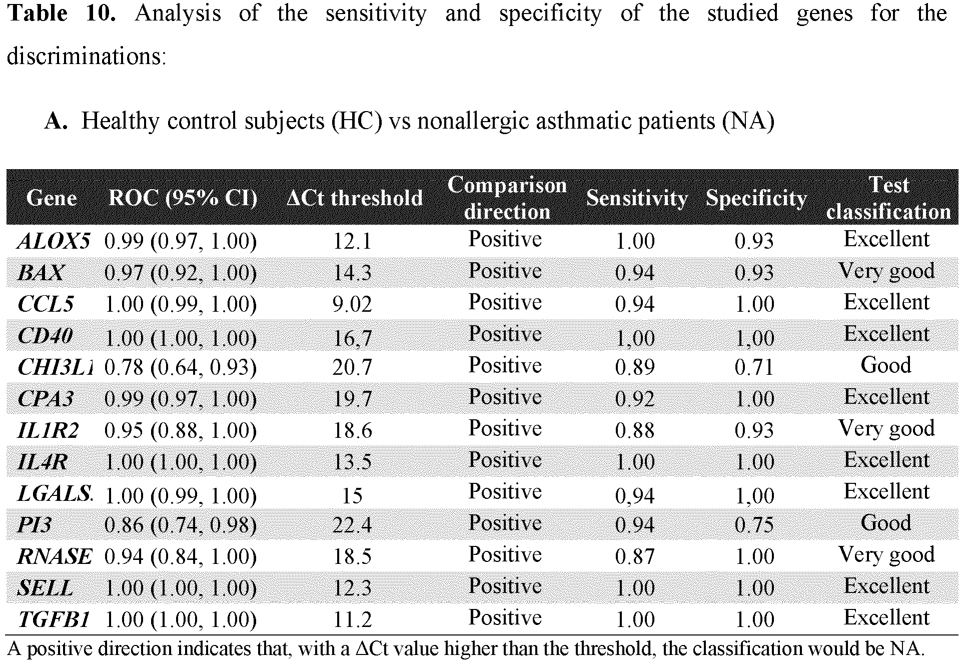

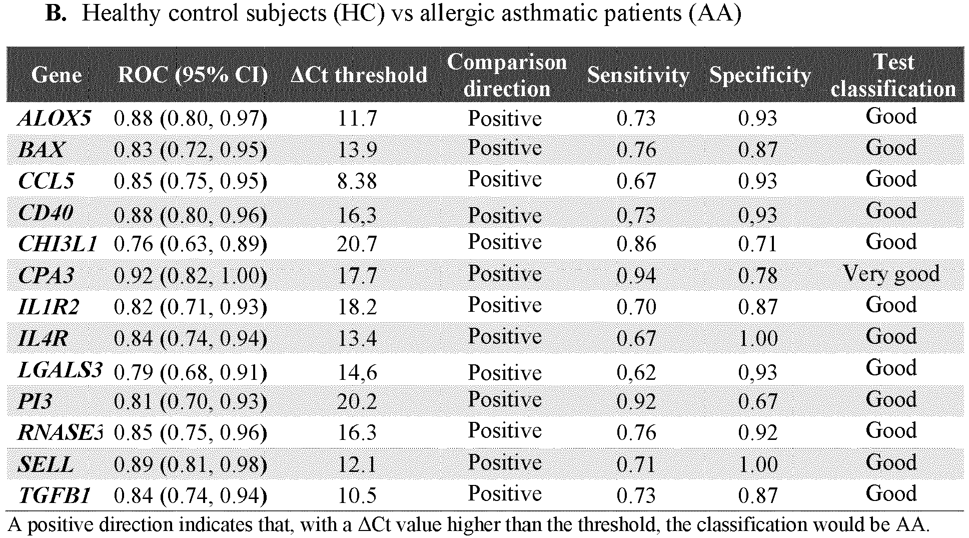

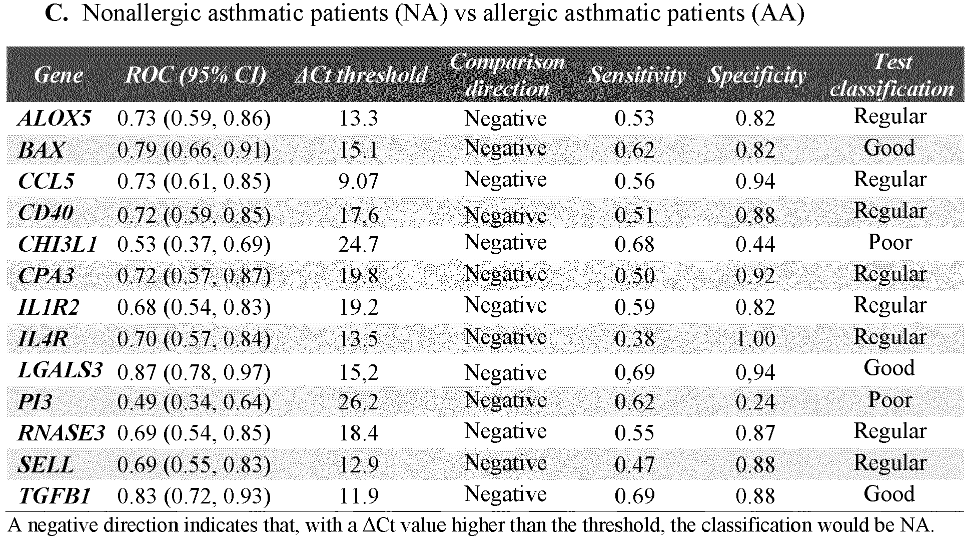

### Example 2.4. Potential of the studied genes to discriminate between severities within each clinical group

Since the severity of asthma is stablished based on the treatment and control of the disease, but there are no objective biomarkers to help the diagnosis, we aim to evaluate the ability of the selected genes to separate the patients according to their previously stablished severity. For that, we used the gene expression data obtained at T0. The classification of the genes respecting the AUC obtained is showed in the **Table 11,** while the complete results of the analysis are showed in the **Table 12.**

In the NA patients **(Table 11A),** any gene were classified as excellent or very good for the severity discrimination, but the mild and severe patients were good separated by *CHI3L1* and *IL1R2.* The rest of the genes and comparisons only gave regular results. In the AA group **(Table 11B),** the mild and the moderate patients are mainly separated by *RNASE3,* while the mild and severe are separated by *ALOX5*, *BAX*, *CCL5*, *CD40*, *IL4R*, *SELL* and *TGFB1.* All these genes were classified as very good tests. However, in the moderate vs severe discrimination, the gene with the highest AUC was *CPA3,* classified as good test. These results indicate that these genes are more useful for the severity discrimination of the AA patients, than for the NA.

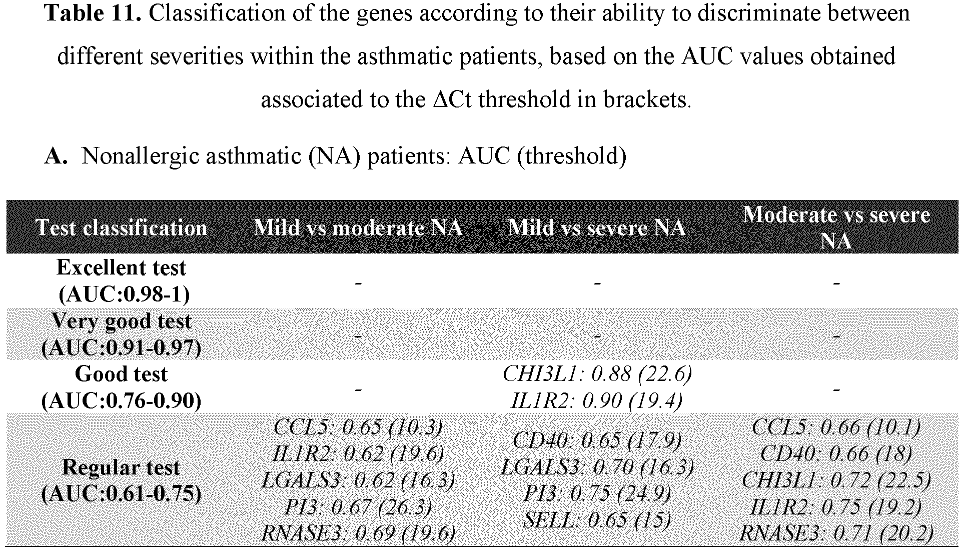

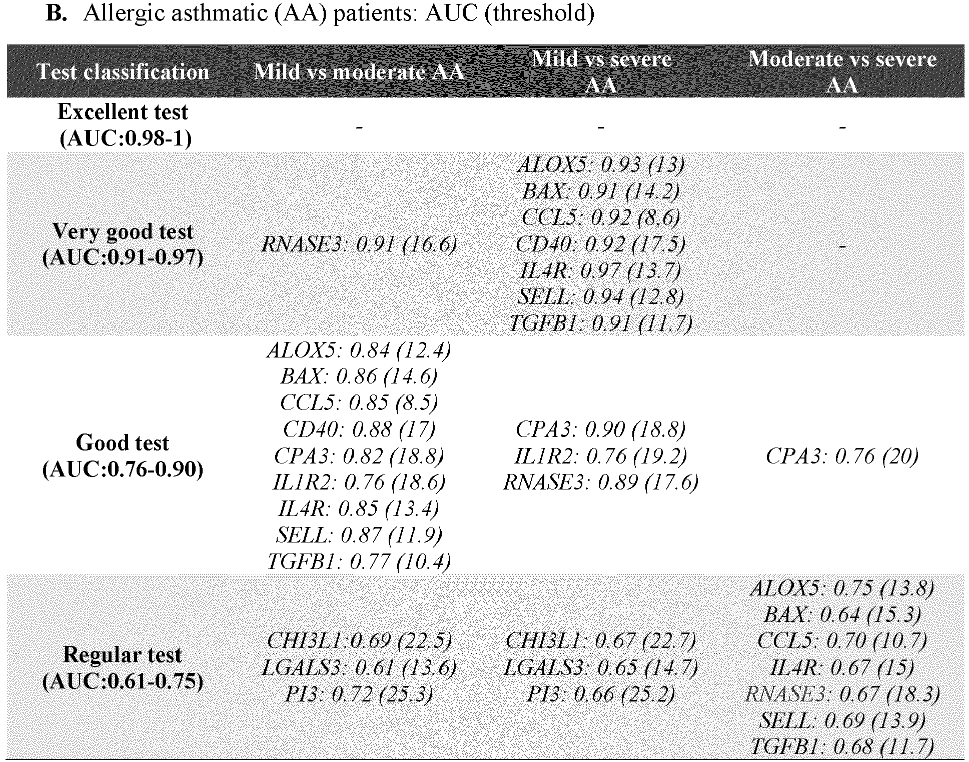

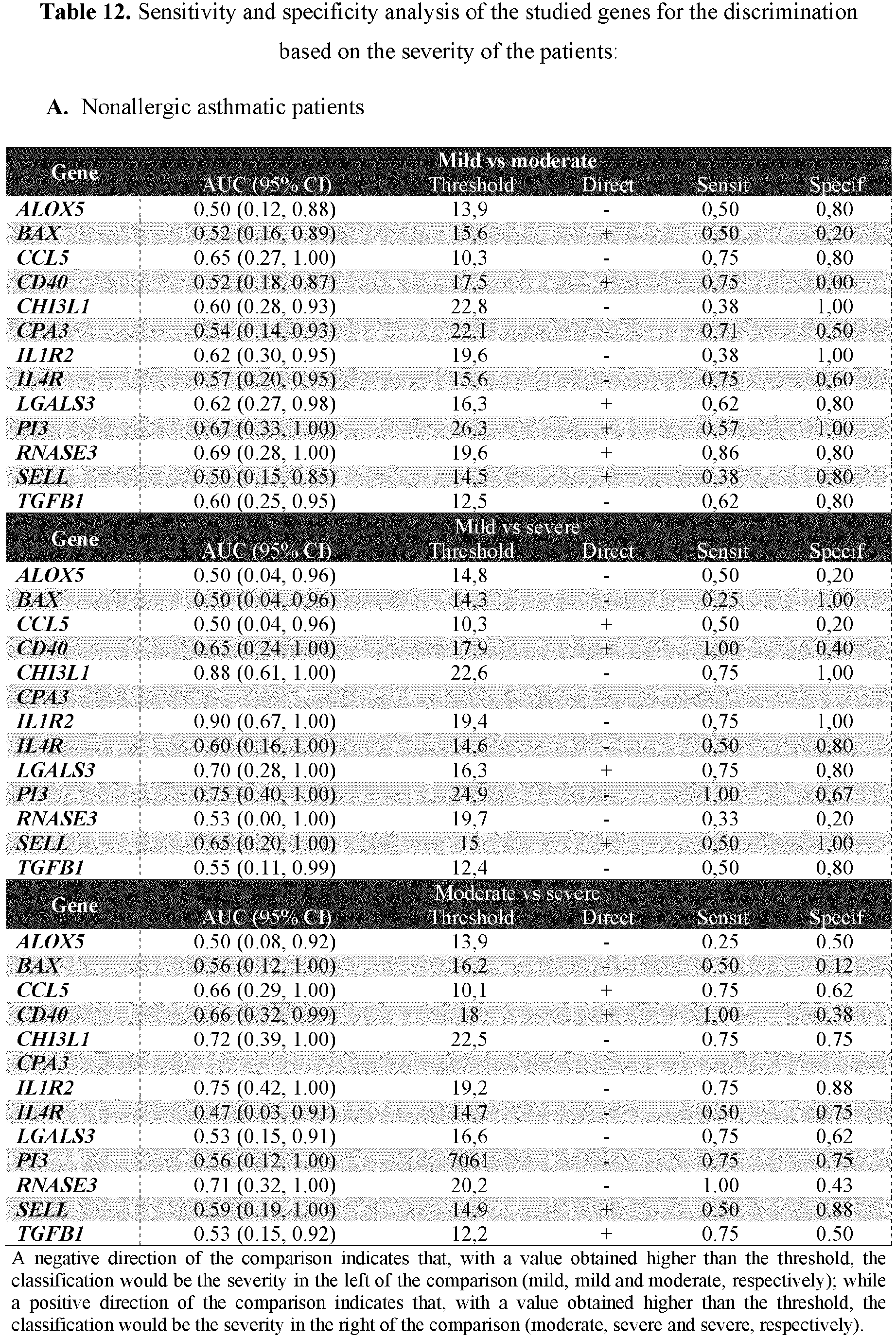

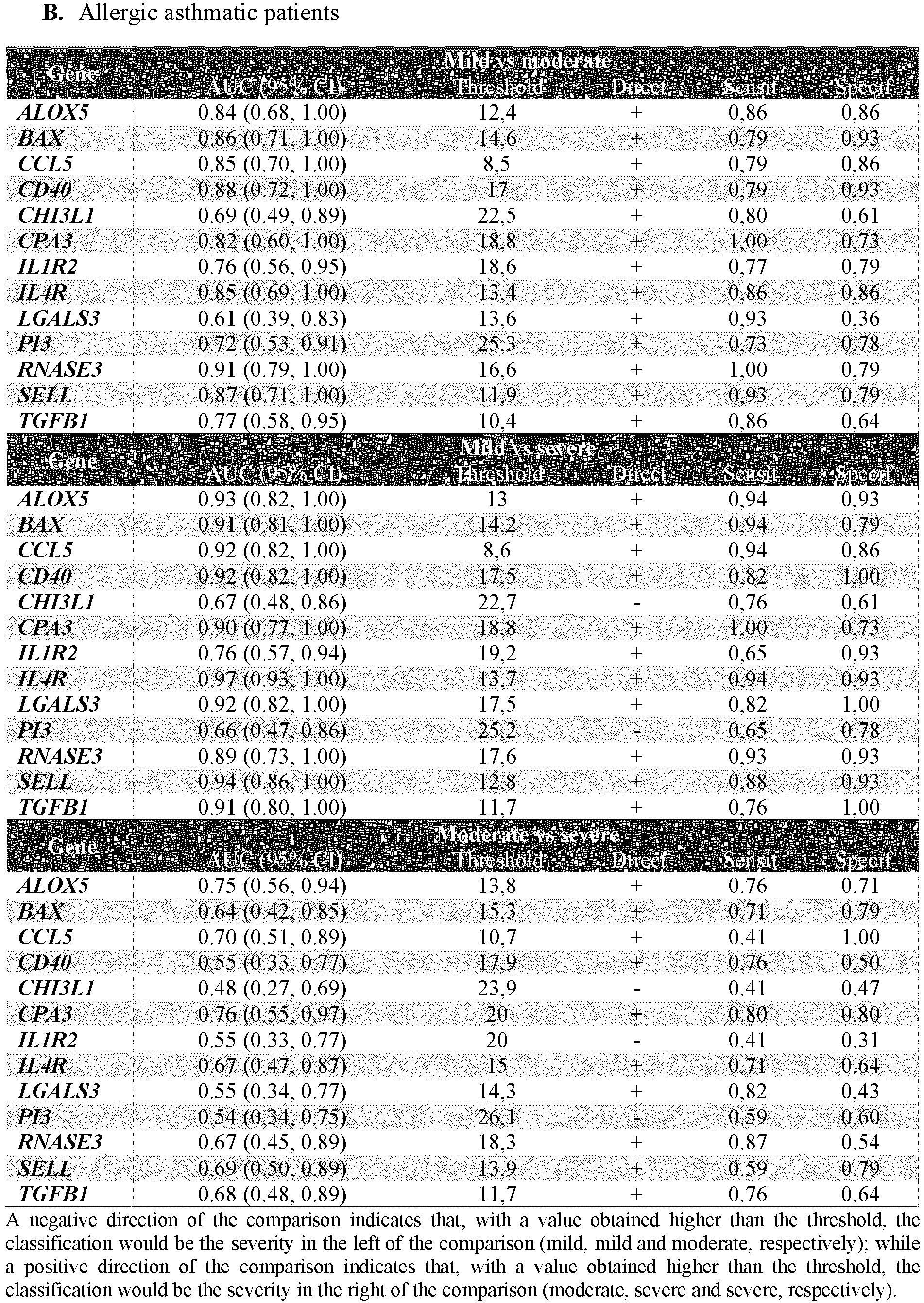

### Example 2.5. Generation of a diagnostic algorithm based on the experimental results

With the aim of simplifying the interpretation of all these information, and generate a useful tool for clinicians, we developed a diagnostic algorithm to guide asthma diagnosis. The algorithm **(****Figure 4****)** is based on the experimental results previously disclosed, and aims to guide the diagnosis, from the discrimination of the three phenotypes studied (HC, NA and AA) to the establishment of the asthma severity, using the least number of genes possible.

Following this algorithm, facing a suspicion of asthma diagnosis would start in the measurement of *CPA3* gene levels in PBMCs isolated from peripheral blood. With a ΔCt obtained lower than 17.7, asthma could be discarded, and other respiratory diseases should be assessed. By contrast, a higher ΔCt would support the asthma suspicions, and with a ΔCt obtained higher than 19.7, nonallergic asthma would be probable. The selection of this gene is based on the ROC curve results obtained for the discrimination of asthma and HC, being the gene with the higher AUC for the separation of AA and HC and presenting an excellent AUC value for the discrimination of NA and HC. This way, with only one gene measured, asthma diagnosis could be performed. In order to differentiate allergic and nonallergic asthma, *LGALS3* and *TGFB1* gene levels should be measured, since those genes were the ones presenting the higher AUC levels for the discrimination of AA and NA. In this case, two genes are necessary since their classification alone was only as "good tests", so the measurement of both genes may help the reduction of mistakes in the asthma classification. With a ΔCt lower than 15.2 and 11.9 (for *LGALS3* and *TGFB1*, respectively), the diagnosis would be of allergic asthma, while higher ΔCt levels would indicate nonallergic asthma. Last, in the severity classification, *RNASE3* and *IL4R* gene levels would help the severity discrimination in AA; while the levels of *IL1R2* and *RNASE3* would be useful in NA.

We conclude that, with the measurement of the expression of only 6 genes in PBMCs samples, asthma diagnosis can be performed, as well as the phenotype and severity classification. However, these results should be confirmed in bigger populations, in order to corroborate them and confirm the given thresholds; as well as measurements in the same population but through longer time periods (5 years, 10 years, and so on) to corroborate the stability of expression. Also, gene expression in whole blood or even protein expression in sera could be relevant to simplify the sample processing.

## Claims

1. *In vitro* method for the diagnosis of allergic asthma or nonallergic asthma in a subject suffering from asthma, or for the differential diagnosis of allergic asthma and nonallergic asthma in a subject suffering from asthma, or for the prognosis of asthma in a subject suffering from asthma, which comprises:
a) assessing the expression level of the *LGALS3* gene in a biological sample obtained from the subject,
b) wherein an increased expression level of the *LGALS3* gene, as compared to a pre-established reference value, is indicative that the subject suffers from allergic asthma, and/or
c) wherein a decreased expression level of the *LGALS3* gene, as compared to a pre-established reference value, is indicative that the subject suffers from nonallergic asthma.

2. *In vitro* method, according to claim 1, which comprises:
a) assessing the expression level of the *LGALS3* gene in combination with a gene selected from *TGFB1*, *ALOX5*, *BAX*, *CCL5*, *CD40*, *CHI3L1*, *CPA3*, *IL1R2*, *IL4R*, *PI3*, *RNASE3* or *SELL* in a biological sample obtained from the subject,
b) wherein an increased expression level of the *LGALS3* gene and of a gene selected from *TGFB1*, *ALOX5*, *BAX*, *CCL5*, *CD40*, *CHI3L1*, *CPA3*, *IL1R2*, *IL4R*, *PI3*, *RNASE3* or *SELL*, as compared to a pre-established reference value, is indicative that the subject suffers from allergic asthma, and/or
c) wherein a decreased expression level of the *LGALS3* gene and of a gene selected from *TGFB1*, *ALOX5*, *BAX*, *CCL5*, *CD40*, *CHI3L1*, *CPA3*, *IL1R2*, *IL4R*, *PI3*, *RNASE3* or *SELL*, as compared to a pre-established reference value, is indicative that the subject suffers from nonallergic asthma.

3. *In vitro* method, according to any of the previous claims, which comprises:
a) assessing the expression level of the genes *LGALS3* and *TGFB1* in a biological sample obtained from the subject,
b) wherein an increased expression level of the genes *LGALS3* and *TGFB1*, as compared to a pre-established reference value, is indicative that the subject suffers from allergic asthma, and/or
c) wherein a decreased expression level of the genes *LGALS3* and *TGFB1*, as compared to a pre-established reference value, is indicative that the subject suffers from nonallergic asthma.

4. *In vitro* method, according to any of the previous claims, which comprises:
a) assessing the expression level of the genes *LGALS3* and *TGFB1* in combination with a gene selected from *ALOX5*, *BAX*, *CCL5*, *CD40*, *CHI3L1*, *CPA3*, *IL1R2*, *IL4R*, *PI3*, *RNASE3* or *SELL* in a biological sample obtained from the subject,
b) wherein an increased expression level of the genes *LGALS3*, *TGFB1* and a gene selected from *ALOX5*, *BAX*, *CCL5*, *CD40*, *CHI3L1*, *CPA3*, *IL1R2*, *IL4R*, *PI3*, *RNASE3* or *SELL*, as compared to a pre-established reference value, is indicative that the subject suffers from allergic asthma, and/or
c) wherein a decreased expression level of the genes *LGALS3*, *TGFB1* and a gene selected from *ALOX5*, *BAX*, *CCL5*, *CD40*, *CHI3L1*, *CPA3*, *IL1R2*, *IL4R*, *PI3*, *RNASE3* or *SELL*, as compared to a pre-established reference value, is indicative that the subject suffers from nonallergic asthma.

5. *In vitro* method, according to any of the previous claims, for the prognosis of allergic asthma or for the differential diagnosis of mild or moderate allergic asthma and severe allergic asthma, which further comprises:
a) assessing the expression level of the *ALOXS* gene, in a biological sample obtained from the subject,
b) wherein an increased expression level of the *ALOXS* gene, as compared to a pre-established reference value, is indicative that the subject suffers from mild or moderate allergic asthma, and/or
c) wherein a decreased expression level of the *ALOXS* gene, as compared to a pre-established reference value, is indicative that the subject suffers from severe allergic asthma.

6. *In vitro* method, according to the previous claim, which further comprises:
a) assessing the expression level of one or more of the genes *BAX*, *CCL5*, *CD40*, *CHI3L1*, *CPA3*, IL4R, *LGALS3*, *PI3*, *RNASE3*, *SELL* or *TGFB1* in a biological sample obtained from the subject,
b) wherein an increased expression level of the genes *BAX*, *CCL5*, *CD40*, *CPA3*, IL4R, *LGALS3*, *RNASE3*, *SELL* or *TGFB1*, and/or a decreased expression level of the genes *CHI3L1* or *PI3*, as compared to a pre-established reference value, is indicative that the subject suffers from mild or moderate allergic asthma, and/or
c) wherein a decreased expression level of the genes *BAX*, *CCL5*, *CD40*, *CPA3*, IL4R, *LGALS3*, *RNASE3*, *SELL* or *TGFB1*, and/or an increased expression level of the genes *CHI3L1* or *PI3*, as compared to a pre-established reference value, is indicative that the subject suffers from severe allergic asthma.

7. *In vitro* method, according to any of the previous claims, for the prognosis of allergic asthma or for the differential diagnosis of mild allergic asthma and moderate allergic asthma, which further comprises:
a) assessing the expression level of the *RNASE3* gene in a biological sample obtained from the subject,
b) wherein an increased expression level of the *RNASE3* gene, as compared to a pre-established reference value, is indicative that the subject suffers from mild allergic asthma, and/or
c) wherein a decreased expression level of the *RNASE3* gene, as compared to a pre-established reference value, is indicative that the subject suffers from moderate allergic asthma.

8. *In vitro* method, according to the previous claim, which further comprises:
a) assessing the expression level of one or more of the genes *ALOX5*, *BAX*, *CCL5*, *CD40*, *CHI3L1*, *CPA3*, *IL1R2*, *IL4R*, *LGALS3*, *PI3*, *SELL* or *TGFB1* in a biological sample obtained from the subject,
b) wherein an increased expression level of the genes *ALOX5*, *BAX*, *CCL5*, *CD40*, *CHI3L1*, *CPA3*, *IL1R2*, *IL4R*, *LGALS3*, *PI3*, *SELL* or *TGFB1*, as compared to a pre-established reference value, is indicative that the subject suffers from mild allergic asthma, and/or
c) wherein a decreased expression level of the genes *ALOX5*, *BAX*, *CCL5*, *CD40*, *CHI3L1*, *CPA3*, *IL1R2*, *IL4R*, *LGALS3*, *PI3*, *SELL* or *TGFB1*, as compared to a pre-established reference value, is indicative that the subject suffers from moderate allergic asthma.

9. *In vitro* method, according to any of the claims 1 to 4, for the prognosis of nonallergic asthma or for the differential diagnosis of mild or moderate nonallergic asthma and severe nonallergic asthma, which comprises:
a) assessing the expression level of the *IL1R2* gene in a biological sample obtained from the subject,
b) wherein a decreased expression level of the *IL1R2* gene, as compared to a pre-established reference value, is indicative that the subject suffers from mild or moderate nonallergic asthma, and/or
c) wherein an increased expression level of the *IL1R2* gene, as compared to a pre-established reference value, is indicative that the subject suffers from severe nonallergic asthma.

10. *In vitro* method, according to the previous claim, which further comprises:
a) assessing the expression level of one or more of the genes *ALOX5*, *BAX*, *CCL5*, *CD40*, *CHI3L1*, *IL4R*, *PI3*, *RNASE3* or *SELL* in a biological sample obtained from the subject,
b) wherein an increased expression level of the genes *CCL5*, *CD40* or *SELL*, and/or a decreased expression level of the genes *ALOX5*, *BAX*, *CHI3L1*, *IL4R*, *PI3* or *RNASE3*, as compared to a pre-established reference value, is indicative that the subject suffers from mild or moderate nonallergic asthma, and/or
c) wherein a decreased expression level of the genes *CCL5*, *CD40* or *SELL*, and/or an increased expression level of the genes *ALOX5*, *BAX*, *CHI3L1*, *IL4R*, *PI3* or *RNASE3*, as compared to a pre-established reference value, is indicative that the subject suffers from severe nonallergic asthma.

11. *In vitro* method, according to any of the claims 1, 2, 3, 4, 9 or 10, for the prognosis of nonallergic asthma or for the differential diagnosis of mild nonallergic asthma and moderate nonallergic asthma, which further comprises:
a) assessing the expression level of the *RNASE3* gene in a biological sample obtained from the subject,
b) wherein an increased expression level of the *RNASE3* gene, as compared to a pre-established reference value, is indicative that the subject suffers from mild nonallergic asthma, and/or
c) wherein a decreased expression level of the *RNASE3* gene, as compared to a pre-established reference value, is indicative that the subject suffers from moderate nonallergic asthma.

12. *In vitro* method, according to the previous claim, which further comprises:
a) assessing the expression level of one or more of the genes *ALOX5*, *BAX*, *CCL5*, *CD40*, *CHI3L1*, *CPA3*, *IL1R2*, *IL4R*, *LGALS3*, *PI3*, *SELL* or *TGFB1* in a biological sample obtained from the subject,
b) wherein an increased expression level of the genes *BAX*, *CD40*, *LGALS3*, *PI3* or *SELL*, and/or a decreased expression level of the genes *ALOX5*, *CCL5*, *CHI3L1*, *CPA3*, *IL1R2*, *IL4R* or *TGFB1*, as compared to a pre-established reference value, is indicative that the subject suffers from mild nonallergic asthma, and/or
c) wherein a decreased expression level of the *BAX*, *CD40*, *LGALS3*, *PI3* or *SELL*, and/or an increased expression level of the genes *ALOX5*, *CCL5*, *CHI3L1*, *CPA3*, *IL1R2*, *IL4R* or *TGFB1*, as compared to a pre-established reference value, is indicative that the subject suffers from moderate nonallergic asthma.

13. *In vitro* method, according to any of the previous claims, wherein the biological sample is peripheral blood mononuclear cells, blood, serum, plasma, bronchoalveolar lavage fluid, preferably peripheral blood mononuclear cells.

14. *In vitro* method, according to any of the previous claims, wherein the subject is a mammal, preferably a human.

15. *In vitro* use of the expression level of the *LGALS3* gene, or of the expression level of the *LGALS3* gene in combination with the expression level of a gene selected from *TGFB1*, *ALOX5*, *BAX*, *CCL5*, *CD40*, *CHI3L1*, *CPA3*, *IL1R2*, *IL4R*, *PI3*, *RNASE3* or *SELL*, or of the expression level of the genes *LGALS3* and *TGFB1*, or of the expression level of the genes *LGALS3* and *TGFB1* in combination with the expression level of a gene selected from *ALOX5*, *BAX*, *CCL5*, *CD40*, *CHI3L1*, *CPA3*, *IL1R2*, *IL4R*, *PI3*, *RNASE3* or *SELL*, for the diagnosis of allergic asthma or nonallergic asthma in a subject suffering from asthma, or for the differential diagnosis of allergic asthma and nonallergic asthma in a subject suffering from asthma, or for the prognosis of asthma in a subject suffering from asthma; or *in vitro* use of a kit comprising reagents for the assessment of the expression level of the *LGALS3* gene, or of the expression level of the *LGALS3* gene in combination with the expression level of a gene selected from *TGFB1*, *ALOX5*, *BAX*, *CCL5*, *CD40*, *CHI3L1*, *CPA3*, *IL1R2*, *IL4R*, *PI3*, *RNASE3* or *SELL*, or of the expression level of the genes *LGALS3* and *TGFB1*, *or* of the expression level of the genes *LGALS3* and *TGFB1* in combination with the expression level of a gene selected from *ALOX5*, *BAX*, *CCL5*, *CD40*, *CHI3L1*, *CPA3*, *IL1R2*, *IL4R*, *PI3*, *RNASE3* or *SELL*, for the diagnosis of allergic asthma or nonallergic asthma in a subject suffering from asthma, or for the differential diagnosis of allergic asthma and nonallergic asthma in a subject suffering from asthma, or for the prognosis of asthma in a subject suffering from asthma.
